# EUROPÄISCHE PATENTANMELDUNG

(11) **EP 4 356 893 A1**
(43) Veröffentlichungstag der Anmeldung: **24.04.2024**
(21) Anmeldenummer: 22202860.7
(22) Anmeldetag: 20.10.2022
(51) Int. Cl.: A61K 6/76, A61K 6/77

(54) **LAGERSTABILE SELBSTHAFTENDE KOMPOSITZEMENTE MIT GUTER TRANSPARENZ UND GUTER RÖNTGENOPAZITÄT**

(71) Anmelder: Ivoclar Vivadent AG, 9494 Schaan (LI)
(72) Erfinder: GIANASMIDIS, Alexandros, 9436 Balgach (CH); CATEL, Yohann, 9475 Sevelen (CH); MOSZNER, Norbert, 9495 Triesen (LI); SIEGRIST, Silke, 66399 Mandelbachtal (DE); BUDIMAN, Ali, 66117 Saarbrücken (DE); BECKER-WILLINGER, Carsten, 66130 Saarbrücken (DE); TWARDOCH, Marek, 57200 Sarreguemines (FR)
(74) Vertreter: Uexküll & Stolberg

(57) **Zusammenfassung**

Radikalisch polymerisierbare Zusammensetzung, die mindestens ein polyfunktionelles radikalisch polymerisierbares Monomer ohne Säuregruppe, mindestens ein säuregruppenhaltiges radikalisch polymerisierbares Monomer und/oder mindestens ein saures Oligomer, mindestens einen Fluoroaluminiumsilikatglasfüllstoff und/oder röntgenopaken Glasfüllstoff und mindestens einen Initiator für die radikalische Polymerisation enthält, wobei der Fluoroaluminiumsilikatglasfüllstoff und/oder röntgenopake Glasfüllstoff mit einem Kieselsäure(hetero)polykondensat beschichtet ist. Die Zusammensetzung zeichnet sich durch eine hohe Stabilität aus und eignet sich besonders als Dentalwerkstoff.

## Beschreibung

Die vorliegende Erfindung betrifft radikalisch polymerisierbare selbsthaftende Komposite mit guter Transparenz und Röntgenopazität, die sich durch eine große Lagerstabilität auszeichnen und sich besonders als Dentalwerkstoffe eignen, insbesondere als dentale Zemente, Füllungskomposite oder Verblendmaterialen sowie zur Herstellung von Inlays, Onlays, oder Kronen.

Komposite werden im Dentalbereich hauptsächlich zur Herstellung von direkten und indirekten Füllungen, d.h. als direkte und indirekte Füllungskomposite, sowie als Zemente eingesetzt. Die polymerisierbare organische Matrix der Komposite besteht meistens aus einer Mischung von Monomeren, Initiator-Komponenten und Stabilisatoren. Als Monomere werden gewöhnlich Mischungen von Dimethacrylaten eingesetzt, die auch monofunktionelle und funktionalisierte Monomere enthalten können. Häufig eingesetzte Dimethacrylate sind 2,2-Bis[4-(2-hydroxy-3-methacryloyloxy-propyl)phenyl]propan (Bis-GMA), 1,6-Bis-[2-methacryloyloxyethoxycarbonylamino]-2,2,4-trimethylhexan (UDMA), die eine hohe Viskosität haben und Polymerisate mit guten mechanischen Eigenschaften und geringem Polymerisationsschrumpf ergeben. Als reaktive Verdünner finden vor allem Triethylenglycoldimethacrylat (TEGDMA), 1,10-Decandioldimethacrylat (D₃MA) oder Bis(3-methacryloyloxymethyl)-tricyclo-[5.2.1.0^{2,6}]decan (DCP) Anwendung. Monofunktionelle Methacrylate, wie z.B. p-Cumylphenoxyethylenglycolmethacrylat (CMP-1E), eignen sich ebenfalls zur Reduzierung der Viskosität und bewirken darüber hinaus eine Verringerung der Netzwerkdichte und einen erhöhten Doppelbindungsumsatz.

Zur Herstellung selbsthaftender Komposite werden stark saure Haftmonomere eingesetzt, wie z.B. 10-Methacryloyloxydecyldihydrogenphosphat (MDP), das die Zahnhartsubstanz ätzt und durch Ionenbeziehung eine Haftung auf Schmelz/Dentin bewirkt. Haftmonomere verleihen Kompositen selbsthaftende Eigenschaften und ermöglichen damit den Einsatz der Komposite ohne eine Vorbehandlung der Zahnhartsubstanz mit einem Schmelz-Dentin-Adhäsiv, was deren Verwendung besonders attraktiv macht.

Neben der organischen Matrix enthalten Komposite einen oder mehrere Füllstoffe, die meist mit einem polymerisationsfähigen Haftvermittler, wie z.B. 3-Methacryloyloxypropyltrimethoxysilan, oberflächenmodifiziert sind. Füllstoffe verbessern die mechanischen Eigenschaften (Festigkeit, Elastizitätsmodul, Abrasionsfestigkeit) und die Verarbeitungseigenschaften (Pastenkonsistenz, Stopfbarkeit) der Werkstoffe und verleihen ihnen Röntgenopazität.

Problematisch ist, dass saure Haftmonomere mit Füllstoffen häufig nachteilige Wechselwirkungen eingehen. Beispielsweise werden die sauren Haftmonomere durch die Bildung unlöslicher Salze an die Oberfläche der Füllstoffe gebunden, oder sie bilden bei der Lagerung mit aus den Füllstoffen herausgelösten Ionen schwerlösliche Salze. Dies führt zu einer deutlichen Verringerung der Haftmonomerkonzentration in der Harzmatrix, was mit einer Verminderung oder sogar einem Verlust der Hafteigenschaften verbunden ist. Komposite mit sauren Haftmonomeren haben daher nur eine begrenzte Lagerstabilität.

Die Aushärtung methacrylatbasierender Dentalmaterialien erfolgt durch radikalische Polymerisation, wobei je nach Anwendungsgebiet radikalische Photoinitiatoren, thermische Initiatoren oder Redox-Initiatorsysteme eingesetzt werden. Dualhärtende Systeme enthalten eine Kombination von Photoinitiatoren und Redoxinitiatoren.

Kompositzemente enthalten in der Regel Redoxsysteme, weil diese auch dann eine ausreichende Härtung gewährleisten, wenn eine Lichthärtung wegen einer unzureichenden Durchstrahlung nicht möglich ist. Meist werden Redoxinitiatorsysteme eingesetzt, die auf einer Mischung von Dibenzoylperoxid (DBPO) mit tertiären aromatischen Aminen, wie z.B. N,N-Diethanol-p-toluidin (DEPT), N,N-Dimethyl-sym.-xylidin (DMSX) oder N,N-Diethyl-3,5-di-tert.-butylanilin (DABA) basieren. Da die Radikalbildung bei DBPO/Amin-basierenden Redoxinitiatorsystemen durch starke Säuren und damit auch durch stark saure Haftmonomere sehr beeinträchtigt wird, werden bevorzugt Cumolhydroperoxid-haltige Redoxinitiatorsysteme in Kombination mit Thioharnstoffen, wie z.B. Acetylthioharnstoff, eingesetzt.

Um eine ausreichende Lagerstabilität der Redoxinitiatoren zu gewährleisten, werden Redox-Initiatorsystem-basierende Materialien meist als sog. 2-Komponenten-Systeme (2K) eingesetzt, wobei das Oxydationsmittel (Peroxid- oder Hydroperoxid) und das Reduktionsmittel (Amine, Sulfinsäuren, Barbiturate, Thioharnstoffe etc.) in räumlich getrennte Komponenten eingearbeitet werden. Diese werden erst kurz vor der Anwendung miteinander gemischt. Zum Mischen werden zunehmend Doppelschubspritzen verwendet, die getrennte zylindrische Kammern zur Aufnahme der Komponenten aufweisen. Die Komponenten werden mit zwei miteinander verbundenen Kolben gleichzeitig aus den Kammern herausgedrückt und in einer Düse miteinander gemischt. Um möglichst homogene Mischungen zu erhalten, ist es vorteilhaft, die Komponenten in etwa gleich großen Volumenanteilen miteinander zu mischen.

Herkömmliche Befestigungszemente, wie z.B. ZnO-Eugenol-Zemente, Zinkphosphat-Zemente, Glasionomer-Zemente (GIZ) und harzmodifizierte Glasionomer-Zemente (Resin Modified Glass Ionomer Cements: RMGI), eigenen sich nicht zur Anwendung mit Doppelschubspritzen, weil sie eine Pulverkomponente enthalten, was ein Mischen der Komponenten erheblich erschwert. Außerdem haben Glasionomerzemente nur eine geringe Transparenz und relativ schlechte mechanische Eigenschaften.

Konventionelle Glasionomer-Zemente (GIZ) enthalten als flüssige Komponente eine wässrige Lösung einer hochmolekularen Polyacrylsäure (PAA, zahlenmittlere Molmasse größer 30 000 g/mol) oder eines Copolymers vergleichbarer Molmasse aus Acrylsäure und Itaconsäure und als Pulverkomponente ein Calcium-Fluor-Aluminium-Glas. Sie härten nach dem Mischen der Komponenten durch eine rein ionische lonomerbildung aus.

Harzmodifizierte Glasionomer-Zemente (Resin Modified Glass Ionomer Cements: RMGI) enthalten zusätzlich hydrophile Monomere, wie z.B. 2-Hydroxyethylmethacrylat (HEMA). Ihre Aushärtung erfolgt sowohl durch eine Säure-Base-Reaktion als auch durch radikalische Polymerisation. Sie zeichnen sich gegenüber herkömmlichen GIZ durch eine verbesserte Biegefestigkeit aus.

Die US 2015/0013568 A1 offenbart die Beschichtung dentaler Barium- und Strontiumglasfüllstoffe mit einem säureresistenten Oxid, beispielsweise mit SiO₂-Pulver, zur Verbesserung der Säureresistenz der Füllstoffe. Hierbei sollen dichte Glaspartikel erhalten werden.

Die WO 2013/156185 A1 offenbart wasserlösliche Oligomere, die durch Hydrolyse und Kondensation von Aminosilanen und anschließende Umsetzung der Reaktionsprodukte mit einem Acrylsäureanhydrid erhalten werden. Bei der direkten Umsetzung von Aminosilanen mit Acrylsäureanhydrid sollen unerwünschte Umesterungsprodukte entstehen, die eine aufwendige Aufreinigung der Produkte erforderlich machen. Die Bildung von Umesterungsprodukten soll durch vorherige Hydrolyse und Kondensation der Aminosilane zu Oligomeren vermieden werden. Die Oligomere sollen sich unter anderem als Haftvermittler oder zur Beschichtung von Füllstoffen eignen, beispielsweise in Zahnabformmassen und Dentalkunststoffmassen.

Der Erfindung liegt die Aufgabe zugrunde, selbsthaftende dentale Komposite mit guter Transparenz, guten mechanischen Eigenschaften und hoher Röntgenopazität bereitzustellen, die sich als 2-Komponentensysteme gut mit Doppelschubspritzen mischen und applizieren lassen. Die Komposite sollen sich insbesondere als dentale Befestigungszemente eignen und eine hohe Lagerstabilität aufweisen.

Diese Aufgabe wird durch füllstoffhaltige, radikalisch polymerisierbare Zusammensetzungen gelöst, die mindestens ein radikalisch polymerisierbares Monomer, mindestens ein saures, radikalisch polymerisierbares Monomer, mindestens einen Fluoroaluminiumsilikatglasfüllstoff (FAS-Füllstoff) und/oder röntgenopaken Glasfüllstoff und mindestens einen Initiator für die radikalische Polymerisation enthalten. Die Zusammensetzungen sind dadurch gekennzeichnet, dass der Füllstoff mit einem Kieselsäure(hetero)polykondensat beschichtet ist. Die Bezeichnung Kieselsäure(hetero)polykondensat steht für Kieselsäurepolykondensat oder Kieselsäureheteropolykondensat. Es wurde überraschend gefunden, dass die Beschichtung des Füllstoffs mit einem Kieselsäure(hetero)polykondensat eine deutliche Erhöhung der Lagerstabilität der Zusammensetzungen bewirkt, ohne die sonstigen Eigenschaften zu verschlechtern. Füllstoffhaltige Zusammensetzungen werden als Komposite bezeichnet.

Das Beschichten des Füllstoffs erfolgt vorzugsweise durch eine Sol-Gel-Verfahren. Hierzu wird durch hydrolytische Kondensation einer oder mehrerer kondensierbarer Verbindungen des Siliciums und gegebenenfalls anderer Elemente aus der Gruppe Bor, Aluminium, Titan oder Phosphor und/oder von den voranstehend genannten Verbindungen abgeleiteten Vorkondensaten ein Sol hergestellt, mit dem Füllstoff in Kontakt gebracht und der Füllstoff anschließend getrocknet.

Bevorzugte hydrolytisch kondensierbare Verbindungen des Siliciums sind Silane der allgemeinen Formel I:

SiXₐR₄₋ₐ Formel I

in der die Variablen die folgenden Bedeutungen haben:
- R =: C₁-C₂₀-Alkyl oder C₂-C₁₀-Alkenyl, wobei Alkyl- und Alkenyl-Reste eine oder mehrere O- oder S-Atome, Ester-, Amid-, Amino- oder Urethan-Gruppen enthalten können, C₆-C₁₄-Aryl oder C₇-C₁₅-Arylalkyl-,
- X =: Halogen, Hydroxy, C₁-C₁₀-Alkoxy-, C₁-C₆-Acyloxy;
- a =: 2, 3 oder 4, vorzugsweise 3 oder 4.

Wenn mehrere R oder X-Reste vorhanden sind, können diese verschieden oder vorzugsweise gleich sein.

Unter Arylalkylgruppen werden Kohlenwasserstoffgruppen verstanden, die mindestens einen aromatischen und mindestens einen nichtaromatischen Rest enthalten, wie zum Beispiel eine -CH₂-CH₂-Ph oder eine -Ph-CH₃-Gruppe mit Ph = Phenyl.

Besonders bevorzugt sind Silane der Formel I in der mindestens eine und vorzugsweise alle Variablen eine der folgenden Bedeutungen haben:
- R =: C₁-C₁₀-Alkyl oder C₂-C₈-Alkenyl, wobei Alkyl- und Alkenyl-Reste ein oder mehrere O-Atome oder Estergruppen enthalten können, Phenyl oder C₇-C₁₅-Arylalkyl;
- X =: Hydroxy, C₁-C₅-Alkoxy-, C₁-C₃-Acyloxy;
- a =: 3 oder 4.

Die Silane der Formel I sind über die Reste X hydrolytisch kondensierbar; damit wird ein anorganisches Netzwerk mit Si-O-Si-Einheiten aufgebaut. Enthalten die Reste R radikalisch polymerisierbare Gruppen, so kann darüber auch zusätzlich ein organisches Netzwerk aufgebaut werden.

Ganz besonders bevorzugt sind Silane der Formel I in der die Variablen die folgenden Bedeutungen haben:
- R: Methyl, Ethyl, n-Propyl, i-Propyl, n-Butyl, s-Butyl, n-Pentyl, Vinyl, (Meth)acryl (H₂C=C(CH₃)-COO-), Allyl, Phenyl oder Naphthyl, besonders bevorzugt sind Ethyl, n-Propyl, i-Propyl und am meisten bevorzugt Methyl oder 3-(Meth)-acryloyloxypropyl;
- X: Methoxy, Ethoxy, n-Propoxy, i-Propoxy, n-Butoxy, F, Cl, Br, besonders bevor-zugt Cl und am meisten bevorzugt Ethoxy;
- a: 3 oder 4.

Erfindungsgemäß sind Silane der Formel SiX₄ (a = 4) besonders bevorzugt, wobei Si(OCH₃)₄ und Si(OC₂H₅)₄ ganz besonders bevorzugt sind. Silane der Formel SiX₄ ergeben eine besonders hohe Netzwerkdichte. Sie werden vorzugsweise in einer Menge von 40 bis 100 Gew.-%, vorzugsweise 50 bis 100 Gew.-% und besonders bevorzugt 60 bis 95 Gew.-% eingesetzt, bezogen auf die Gesamtmenge an Silanen der Formel I.

Weiter bevorzugt sind Silane der Formel I, die mindestens eine radikalisch polymerisierbare Gruppe enthalten, wobei 3-Methacryloyloxypropyltrimethoxysilan besonders bevorzugt ist. Die radikalisch polymerisierbare Gruppe ermöglicht einen kovalenten Verbund der beschichteten Füllstoffe mit der radikalisch polymerisierbaren Matrix bei der Härtung der erfindungsgemäßen Werkstoffe. Silane mit radikalisch polymerisierbaren Gruppen werden vorzugsweise in einer Menge von 0 bis 10 Gew.-%, vorzugsweise 0 bis 8 Gew.-% und besonders bevorzugt 1 bis 5 Gew.-% eingesetzt, bezogen auf die Gesamtmenge an Silanen der Formel I.

Neben dem oder den Silanen der Formel I können eine oder mehrere weitere hydrolytisch kondensierbare Verbindungen der Elemente Al, Ti, B oder P zur Beschichtung der Füllstoffe eingesetzt werden. Bevorzugt sind Verbindungen des Al, Ti und B. Bevorzugte Aluminiumverbindungen sind Aluminium-sek-butylat und Aluminiumisopropylat. Bevorzugte Titanverbindungen sind Ti(OC₂H₅)₄, Ti(OC₃H₇)₄, Ti(O-i-C₃H₇)₄ und Ti(OC₄H₉)₄. Bevorzugte Borverbindungen sind B(OCH₃)₃ und B(OC₂H₅)₃. Durch die Verwendung weiterer hydrolytisch kondensierbarer Verbindungen können die Eigenschaften der Beschichtung, wie z.B. Härte und Abriebsfestigkeit, gezielt beeinflusst werden. Es können eine oder mehrere zusätzliche Verbindungen verwendet werden. Diese werden vorzugsweise in einer Gesamtmenge von 0 bis 20 Gew.-%, vorzugsweise 0 bis 15 Gew.-% und besonders bevorzugt 0 bis 10 Gew.-% eingesetzt, bezogen auf die Gesamtmenge an Silanen der Formel I. Metallalkoxide, beispielsweis von Al oder Ti, können auch in komplexierter Form eingesetzt werden, z.B. in Form der Umsetzungsprodukte von Al- oder Ti-Alkoxiden mit (Meth)acrylsäure oder 2-Acetoacetoxyethylmethacrylat. Der Anteil an Silanen der Formel I beträgt vorzugsweise 80 bis 100 Gew.-%, besonders bevorzugt 85 bis 100 Gew.-% und ganz besonders bevorzugt 90 bis 100 Gew.-%, bezogen auf die Gesamtmenge an hydrolytisch kondensierbaren Verbindungen.

Das oder die Silane der Formel I und gegebenenfalls andere hydrolytisch kondensierbaren Verbindungen werden in Gegenwart von Wasser, vorzugsweise mindestens der zur Hydrolyse stöchiometrisch erforderlichen Wassermenge, und gegebenenfalls eines Kondensationskatalysators und/oder eines Lösungsmittels hydrolysiert und kondensiert. Hierbei entstehen Polykondensate bzw. Heteropolykondensate. Anstelle der hydrolytisch kondensierbaren Verbindungen können auch davon abgeleitete Vorkondensate eingesetzt werden. Vorkondensate sind Umsetzungsprodukte der Alkoxysilane und/oder Halogensilane und ggf. der Metallalkoxide mit Wasser unter teilweiser- oder vollständiger Abspaltung der Alkohole bzw. Halogenwasserstoffe. Im Falle der Alkoxysilane entstehen Silanole als Vorkondensate.

Vorzugsweise führt man die hydrolytische Kondensation so durch, dass man den zu beschichtenden Füllstoff in dem oder den Silanen der Formel I und ggf. weiteren hydrolytisch kondensierbaren Verbindungen oder vorzugsweise einer Lösung des oder der Silane und ggf. weiterer hydrolytisch kondensierbarer Verbindungen in einem geeigneten Lösungsmittel dispergiert und man dann Wasser zugibt. Die Mischung wird hierbei vorzugsweise gerührt.

Werden ausschließlich Silane eingesetzt, führt man die Hydrolyse vorzugsweise bei Raumtemperatur oder unter leichter Kühlung durch. Vorzugsweise wird ein Hydrolyse- oder Kondensationskatalysators zugesetzt. Hierbei werden Polykondensate erhalten. Die resultierende Mischung (Dispersion) wird vorzugsweise für mehrere Stunden bis einige Tage bei einer Temperatur von -20 bis 150°C, vorzugweise 20 bis 110°C, gerührt. Anschließend wird der Füllstoff abgetrennt, z.B. durch Zentrifugation, dann gegebenenfalls ein oder mehrfach mit einem Lösungsmittel gewaschen und danach getrocknet. Das Trocken kann bei Raumtemperatur oder bei erhöhter Temperatur, vorzugsweise bei 50 bis 60°C, durchgeführt werden. Vorzugsweise wird der Füllstoff in einem Vakuumtrockenschrank getrocknet. Das Trocken kann sich über einen Zeitraum von einigen Stunden bis zu einigen Tagen erstrecken. Vorzugsweise wird bis zur Gewichtskonstanz getrocknet. Die Füllstoffe können nach dem Trocknen einer weiteren Wärmebehandlung unterworfen werden. Diese wird vorzugweise bei einer Temperatur von 20 bis 150°C, besonders bevorzugt von 30 bis 100°C und ganz besonders von 40 bis 80°C durchgeführt. Temperatur und Behandlungszeit werden so gewählt, dass in der Beschichtung evtl. vorhandene organische Gruppen nicht zerstört werden und keine Glasschicht ausgebildet wird.

Werden neben den Silanen der Formel I weitere hydrolytisch kondensierbare Verbindungen zur Beschichtung des Füllstoffs eingesetzt, erfolgt die Zugabe des Wassers vorzugsweise stufenweise bei -20 bis 100°C, vorzugsweise bei 0 bis 30°C. In diesem Fall bilden sich Heteropolykondensate. Die Art und Weise der Wasserzugabe richtet sich vor allem nach der Reaktivität der eingesetzten hydrolytisch kondensierbaren Verbindungen. Die gewünschte Wassermenge kann z.B. portionsweise oder in einer Portion zugeben werden.

Das Wasser wird sowohl bei der Herstellung von Polykondensaten als auch von Heteropolykondensaten vorzugsweise in einer Menge von 0,5 bis 10 Mol Wasser pro Mol hydrolysierbarer Gruppen, besonders bevorzugt in einer Menge von 1 bis 10 Mol Wasser pro Mol hydrolysierbarer Gruppen und ganz besonders bevorzugt in einer Menge von 1,5 bis 5 Mol Wasser pro Mol hydrolysierbarer Gruppen der Silane der Formel I und ggf. weiterer hydrolytisch kondensierbaren Verbindungen zugesetzt. Das Wasser oder Anteile davon können in reiner Form oder als Bestandteil anderer Komponenten, z.B. der Katalysatorlösung, eingebracht werden.

Bevorzugte Lösungsmittel zur Durchführung der hydrolytischen Kondensation sind niedere aliphatische Alkohole, wie Ethanol oder i-Propanol, niedere Dialkylketone, wie Aceton oder Methylisobutylketon, cyclische Ether, wie Tetrahydrofuran oder Dioxan, aliphatische Ether, wie Isopropoxyethanol oder Isopropoxypropanol, Amide, wie Dimethylformamid, und Mischungen davon.

Als Hydrolyse- oder Kondensationskatalysatoren sind organische oder anorganische Säuren, wie Salzsäure, Schwefelsäure, p-Toluolsulfonsäure, Ameisensäure oder Essigsäure bevorzugt. Weiter bevorzugt sind Ammoniumfluorid und organische Basen, insbesondere Amine, wie n-Propylamin, Diethyl- oder Triethylamin sowie Aminosilane, wie z.B. 3-Aminopropyltrimethoxysilan.

Die Kondensationszeit hängt von den jeweiligen Ausgangskomponenten, deren Mengenanteilen, dem gegebenenfalls verwendeten Katalysator, der Reaktionstemperatur etc. ab. Vorzugsweise liegt die Reaktionszeit in einem Bereich von 1 bis 96 h, besonders bevorzugt 2 bis 72 h und ganz besonders bevorzugt 3 bis 48 h.

Beim Trocknen der Füllstoffe und Verdampfen des Lösungsmittels führt die Hydrolyse- und Kondensationsreaktion über die Bildung von Sol-Partikeln zur Bildung eines Gelfilms, der sich im Verlauf der Trocknung weiter vernetzt und ein dichtes anorganisches Netzwerk auf der Füllstoffoberfläche ausbildet. Die Eigenschaften der Beschichtung, wie z.B. Festigkeit, Flexibilität, Haftung, Härte, Brechungsindex und Dichtigkeit, lassen sich durch die Auswahl der Silane und der weiteren hydrolytisch kondensierbaren Komponenten gezielt einstellen und an den jeweiligen Verwendungszweck anpassen. Beispielsweise kann über die Funktionalität der Silane der Formel I, die zwischen 4, z.B. in Tetraethoxysilan (TEOS), und 2, z.B. in Dialkoxysilan, variieren kann, die Vernetzungsdichte und damit die Festigkeit, Flexibilität und Dichtigkeit der Beschichtung eingestellt werden. Durch die Wahl der organischen Gruppen der Silane ist eine gezielte Beeinflussung beispielsweise der Flexibilität, Polarität, Benetzung oder Verdickungswirkung der Beschichtung möglich. Silane mit polymerisierbaren Gruppen, wie z.B. in 3-Methacryloyloxypropyltrimethoxysilan, ermöglichen eine zusätzliche Vernetzung der Beschichtung durch radikalische Polymerisation und eine kovalente Einbindung der Füllstoffe in die Polymermatrix. Die Cokondensation mit Metallalkoxiden, wie z.B. Ti- Alkoxid, ermöglicht eine Steuerung der Kinetik der hydrolytischen Kondensation und z.B. eine Erhöhung der Dichte und Härte sowie die Einstellung des Brechungsindexes der Beschichtung.

Der Füllstoff wird vorzugsweise in einer Menge von 1 bis 25 Gew.-%, besonders bevorzugt von 3 bis 20 Gew.-% und ganz besonders bevorzugt von 4 bis 15 Gew.-% in der Hydrolysemischung dispergiert, bezogen auf die Gesamtmasse der Mischung. Erfindungsgemäß sind partikuläre Füllstoffe bevorzugt.

Als zu beschichtende Füllstoffe sind Fluoroaluminiumsilikatglasfüllstoffe (FAS-Füllstoffe) und röntgenopake Glasfüllstoffe bevorzugt.

Besonders bevorzugte röntgenopake Glasfüllstoffe weisen die folgende Zusammensetzung auf (Gew.-%): SiO₂: 20-80; B₂O₃: 5-15; BaO oder SrO: 0-30; Al₂O₃: 5-20; CaO und/oder MgO: 0-20; Na₂O, K₂O, Cs₂O: je 0-10; WO₃: 0-20; La₂O₃: 0-10; ZrO₂: 0-15; P₂O₅: 0-10; Ta₂O₅, Nb₂O₅ oder Yb₂O₃: 0-5 und CaF₂ oder SrF₂: 0-10. Ganz besonders bevorzugt sind röntgenopake Glasfüller mit der Zusammensetzung (Gew.- %): SiO₂: 50-75; B₂O₃: 5-15; BaO oder SrO: 2-30; Al₂O₃: 5-15; CaO und/oder MgO: 0-10 und Na₂O: 0-10.

Besonders bevorzugte FAS-Füller haben die folgende Zusammensetzung (Gew.-%): SiO₂: 20-35; Al₂O₃: 15-35; BaO oder SrO: 0-25; CaO: 0-20; ZnO: 0-15; P₂O₅: 5-20; Fluorid: 3-18. Ganz besonders bevorzugt sind FAS-Füller mit der Zusammensetzung (Gew.-%): SiO₂: 20-30; Al₂O₃: 20-30; BaO oder SrO: 15-15; CaO: 5-18; P₂O₅: 5-15; Na₂O: 2-10; und CaF₂: 5-20.

Alle Prozentangaben beziehen sich auf die Gesamtmasse des Glases, wobei die Komponenten mit Ausnahme von Fluor als Oxide berechnet werden, so wie es bei Gläsern und Glaskeramiken üblich ist, und schließen die Beschichtung nicht ein.

Erfindungsgemäß sind partikuläre FAS-Füller und röntgenopaken Glasfüller mit einer mittleren Partikelgröße von 0,2 bis 20 µm bevorzugt und mit einer mittleren Partikelgröße von 0,4 bis 5 µm besonders bevorzugt. Die Größenangaben schließen die Beschichtung nicht ein. Die Schichtdicke der Beschichtung liegt vorzugweise in einem Bereich von 5 bis 500 nm.

Wenn nicht anders angegeben, handelt es sich bei allen Partikelgrößen hierin um volumengemittelte Partikelgrößen (D50-Werte), d.h. 50 % des Gesamtvolumens, das alle Partikel gemeinsam besitzen, ist in Partikeln enthalten, die einen Durchmesser kleiner als der angegebene Wert aufweisen.

Die Partikelgrößenbestimmung im Bereich von 0,1 µm bis 1000 µm erfolgt vorzugsweise mittels statischer Lichtstreuung (SLS), beispielsweise mit einem statischen Laserstreuungs-Partikelgrößen-Analysator LA-960 (Horiba, Japan) oder mit einem Microtrac S100 Partikelgrößen-Analysator (Microtrac, USA). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße in einem Bereich von 1 nm bis 0,1 µm erfolgt vorzugsweise durch dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25°C, z.B. mit einem Malvern Mastersizer 2000 mit Dispergiereinheit Hydro 2000S (Malvern Instruments, Malvern UK).

Im Fall aggregierter und agglomerierter Partikel kann die Primärteilchengröße anhand von TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf ein 50 A dickes Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Die röntgenopaken Glasfüllstoffe oder FAS-Füllstoffe können vor dem Beschichten mit Säure vorbehandelt werden. Die Säurebehandlung der partikulären FAS- bzw. Glasfüllstoffe erfolgt vorzugsweise dadurch, dass der Füllstoff mit Säure gewaschen wird, besonders bevorzugt durch folgendes Verfahren:
(i) Der partikuläre FAS- bzw. Glasfüllstoff wird in einer wässrigen Lösung einer organischen oder vorzugsweise anorganischen Säure dispergiert. Die Säure wird vorzugsweise in einer Konzentration von 0,1 bis 5 mol/l, besonders bevorzugt von 0,5 bis 3 mol/l eingesetzt.
(ii) Anschließend wird die Dispersion aus Schritt (i) gerührt, vorzugsweise für 0,5 bis 24 h, besonders bevorzugt für 1 bis 5 h.
(iii) Nach dem Rühren in Säurelösung wird der Füllstoff abgetrennt und mit deionisiertem Wasser gewaschen. Hierzu wird er vorzugsweise in Wasser dispergiert und für 1 bis 60 Minuten, besonders bevorzugt 2 bis 20 Minuten gerührt.
(iv) Nach dem Waschen wird der Füllstoff abgetrennt und getrocknet, vorzugsweise in einem Vakuumtrockenschrank im Feinvakuum bei 20 bis 80°C, besonders bevorzugt bei 40 bis 60°C. Der Füllstoff wird vorzugsweise bis zur Gewichtskonstanz getrocknet.
(v) Nach dem Trocknen wird der Füllstoff vorzugsweise einer optionalen Temperung unterworfen. Hierzu wird er vorzugsweise für 2 bis 12 h, besonders bevorzugt 4 bis 6 h, auf eine Temperatur erwärmt, die deutlich unterhalb der Glasübergangstemperatur des Füllstoffs liegt, vorzugsweise 200 bis 500°C und besonders bevorzugt 300 bis 400°C.

Die zur Behandlung der Füllstoffe verwendete Säure wird nach der Säurebehandlung vorzugsweise vollständig von den Füllstoffen entfernt. Die Füllstoffe sind nicht mit einer Säure beschichtet.

Die Säurebehandlung kann ein- oder mehrfach wiederholt werden. Die Schritte (i) und (iii) werden vorzugsweise bei einer Temperatur im Bereich von 5 bis 50°C durchgeführt, besonders bevorzugt bei Raumtemperatur (23°C). Die Temperatur wird jeweils in der Lösung bzw. Dispersion gemessen.

Bevorzugt sind Säuren, die lösliche Salze mit Ca- Al-, Sr- und Ba-Ionen bilden. Besonders bevorzugt sind Ameisensäure, Essigsäure und ganz besonders Salzsäure, und Salpetersäure. Alternativ können auch Säuren eingesetzt werden, die schwerlösliche Salze mit Ca-, Al-, Sr- oder Ba-Ionen bilden, wie z.B. Phosphorsäure, diese sind jedoch weniger bevorzugt. Unter schwerlöslichen Salzen werden Salze mit einer Löslichkeit von unter 0,1 g/l verstanden (in Wasser bei Raumtemperatur). Saure organische Monomere und saure organische Polymere sowie Persäuren und Flusssäure sind erfindungsgemäß als Säuren nicht geeignet.

Der Waschvorgang (iii) wird vorzugsweise 1 bis 5 Mal, besonders bevorzugt 3 Mal wiederholt, so dass die Säure vollständig von dem Füllstoff entfernt wird. Hierzu wird der Füllstoff im Anschluss an Schritt (iii) von dem Wasser abgetrennt und erneut in deionisiertem Wasser dispergiert und gerührt. Das Waschen wird so oft wiederholt, bis der pH-Werts des Wassers im letzten Waschschritt ≥ 5 ist.

Nach dem Waschen kann der Füllstoff einer weiteren Säurebehandlung und Wäsche unterworfen werden. Die Sequenz von Säurebehandlung und Waschen kann ein- oder mehrfach wiederholt werden.

Die erfindungsgemäße Beschichtung der Füllstoffe verhindert, dass signifikante Mengen an Metallionen, insbesondere Al-, Ca-, Ba- oder Sr-Ionen, aus dentalen Füllstoffen, wie röntgenopaken Glasfüllstoffen und FAS-Füllstoffen, in der Harzmischung diffundieren und dort mit sauren Monomeren reagieren. Außerdem verhindert sie, dass saure Monomere an der Füllstoffoberfläche durch die Bildung unlöslicher Salze gebunden werden. Dadurch wird eine Abnahme der Konzentration an sauren Monomeren die der Harzmatrix und eine damit einhergehende Verschlechterung der Haftung vermieden und die Lagerstabilität der Werkstoffe verbessert.

Zusätzlich zu den beschichteten FAS- und röntgenopaken Glasfüllstoffen können die erfindungsgemäßen Zusammensetzungen weitere Füllstoffe enthalten.

Bevorzugte weitere Füllstoffe sind Metalloxide, besonders bevorzugt Mischoxide, die 60 bis 80 Gew.-% SiO₂ und mindestens eines der Metalloxide ZrO₂, Yb₂O₃, ZnO, Ta₂O₅, Nb₂O₅ und/oder La₂O₃, vorzugsweise ZrO₂, Yb₂O₃ und/oder ZnO enthalten, so dass sich die Gesamtmenge auf 100 % ergänzt. Mischoxide wie SiO₂-ZrO₂ sind z.B. durch hydrolytische Cokondensation von Metallalkoxiden zugänglich. Die Metalloxide haben vorzugsweise eine mittlere Partikelgröße von 0,05 bis 10 µm, besonders bevorzugt von 0,1 bis 5 µm. Das oder die Metalloxide werden vorzugsweise ebenfalls auf die oben beschriebene Weise beschichtet.

Weitere bevorzugte zusätzliche Füllstoffe sind pyrogene Kieselsäure oder Fällungskieselsäure mit einer Primärpartikelgröße von 0,01 bis 0,15 µm sowie Quarz- oder Glaskeramikpulver mit einer Partikelgröße von 0,1 bis 15 µm, vorzugweise von 0,2 bis 5 µm, sowie Ytterbiumtrifluorid. Das Ytterbiumtrifluorid hat vorzugsweise eine Partikelgröße von 80 bis 900 nm und besonders bevorzugt 100 bis 300 nm. Diese Füller werden vorzugsweise in einer Menge von 0,1 bis 25 Gew.-%, besonders bevorzugt 0,2 bis 20 Gew.-% und ganz besonders bevorzugt in einer Menge von 0,3 bis 15 Gew.-% eingesetzt, jeweils bezogen auf die Gesamtmasse der Zusammensetzung.

Außerdem sind als weitere Füllstoffe sog. Kompositfüller bevorzugt. Diese werden auch als Isofüller bezeichnet. Hierbei handelt es sich um splitterförmige Polymerisate, die ihrerseits einen Füllstoff enthalten, vorzugsweise pyrogenes SiO₂, Glasfüller und/oder Ytterbiumtrifluorid. Bevorzugt sind Polymerisate auf der Basis von Dimethacrylaten. Zur Herstellung der Isofüller werden der oder die Füllstoffe z.B. in eine Dimethacrylatharzmatrix eingearbeitet, die so erhaltene Kompositpaste anschließend thermisch polymerisiert und dann gemahlen.

Ein erfindungsgemäß bevorzugter Kompositfüller lässt sich beispielsweise durch thermische Härtung einer Mischung von Bis-GMA (8,80 Gew.-%), UDMA (6,60 Gew.- %), 1,10-Decandioldimethacrylat (5,93 Gew.-%), Dibenzoylperoxid + 2,6-Di-tert.-butyl-4-methylphenol (zusammen 0,67 Gew.-%), Glasfüller (mittlere Korngröße 0,4 µm; 53,0 Gew.-%) und YbF₃ (25,0 Gew.-%) und anschließendes Vermahlen des gehärteten Materials auf die gewünschte Korngröße herstellen. Alle Prozentangaben beziehen sich auf die Gesamtmasse des Kompositfüllers.

Zur Verbesserung des Verbundes zwischen Füllstoff und Matrix sind die Füllstoffe vorzugsweise mit methacrylatfunktionalisierten Silanen, wie z.B. 3-Methacryloyloxypropyltrimethoxysilan, oberflächenmodifiziert.

Die erfindungsgemäßen Zusammensetzungen enthalten mindestens ein radikalisch polymerisierbares polyfunktionelles Monomer. Unter polyfunktionellen Monomeren werden Verbindungen mit zwei oder mehr, vorzugsweise 2 bis 4 und insbesondere 2 radikalisch polymerisierbaren Gruppen verstanden. Monofunktionelle Monomere weisen dementsprechend nur eine radikalisch polymerisierbare Gruppe auf. Polyfunktionelle Monomere haben vernetzende Eigenschaften und werden daher auch als Vernetzermonomere bezeichnet. Bevorzugte radikalisch polymerisierbare Gruppen sind (Meth)acrylat-, (Meth)acrylamid- und Vinylgruppen.

Erfindungsgemäß wird zwischen säuregruppenhaltigen Monomeren und Monomeren unterschieden, die keine Säuregruppen enthalten. Die erfindungsgemäßen Zusammensetzungen enthalten mindestens ein Monomer ohne Säuregruppen und mindestens ein Monomer und/oder Oligomer mit Säuregruppen. Erfindungsgemäß sind Zusammensetzungen bevorzugt, die Monomere mit und Monomere ohne Säuregruppe in einem Gewichtsverhältnis von 1:5 bis 1:36, besonders bevorzugt von 1:6 bis 1:25 und ganz besonders bevorzugt von 1:7 bis 1:20 enthalten.

### Monomere ohne Säuregruppe

Bevorzugt sind Zusammensetzungen, die mindestens ein polyfunktionelles (Meth)acrylat enthalten, besonders bevorzugt mindestens ein polyfunktionelles und mindestens ein monofunktionelles Methacrylat und ganz besonders bevorzugt mindestens ein monofunktionelles und mindestens ein difunktionelles Methacrylat.

Bevorzugte monofunktionelle (Meth)acrylate sind Benzyl-, Tetrahydrofurfuryl- oder Isobornyl(meth)acrylat, p-Cumyl-phenoxyethylenglycolmethacrylat (CMP-1E) und 2-([1,1'-Biphenyl]-2-oxy)ethylmethacrylat (MA-836), Tricyclodecanmethyl(meth)-acrylat, 2-(2-Biphenyloxy)ethyl(meth)acrylat. Besonders geeignet sind CMP-1E und MA-836.

Gemäß einer Ausführungsform enthalten die erfindungsgemäßen Zusammensetzungen vorzugsweise mindestens ein funktionalisiertes monofunktionelles (Meth)acrylat. Unter funktionalisierten Monomeren werden solche Monomere verstanden, die neben mindestens einer radikalisch polymerisierbaren Gruppe mindestens eine funktionelle Gruppe tragen, vorzugsweise eine Hydroxylgruppe. Bevorzugte funktionalisierte Mono(meth)acrylate sind 2-Hydroxyethyl(meth)acrylat und Hydroxyethylpropyl(meth)acrylat sowie 2-Acetoxyethylmethacrylat. Besonders bevorzugt ist Hydroxyethylmethacrylat. Die unten genannten säuregruppenhaltigen Monomere sind keine funktionalisierten Monomere im Sinne der Erfindung.

Bevorzugte di- und polyfunktionelle (Meth)acrylate sind Bisphenol-A-dimethacrylat, Bis-GMA (ein Additionsprodukt aus Methacrylsäure und Bisphenol-A-diglycidylether), ethoxy- oder propoxylierte Bisphenol-A-dimethacrylate, wie z.B. das Bisphenol-A-dimethacrylat mit 3 Ethoxygruppen (SR-348c, Sartomer) oder 2,2-Bis[4-(2-methacryloyloxypropoxy)phenyl]propan, Urethane aus 2-(Hydroxymethyl)acrylsäuremethylester und Diisocyanten, wie 2,2,4-Trimethylhexamethylendiisocyanat oder Isophorondiisocyanat, UDMA (ein Additionsprodukt aus 2-Hydroxyethylmethacrylat und 2,2,4-Trimethylhexamethylen-1,6-diisocyanat), Tetramethylxylylendiurethanethylenglykoldi-(meth)acrylat bzw. Tetramethylxylylendiurethan-2-methylethylenglykoldi(meth)acrylat (V380), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryloyloxy-methyl)tricyclo-[5.2.1.0^{2,6}]decan (DCP), Polyethylenglycol- oder Polypropylenglycoldimethacrylate, wie z.B. Polyethylenglycol-200-dimethacrylat oder Polyethylenglycol-400-dimethacrylat (PEG-200- oder PEG-400-DMA) oder 1,12-Dodecandioldimethacrylat. Besonders bevorzugt sind Bis-GMA, UDMA, V-380, Triethylenglycoldimethacrylat (TEGDMA) und PEG-400-DMA (NK-Ester 9G).

Das Monomer Tetramethylxylylendiurethanethylenglykoldi(meth)acrylat bzw. Tetramethylxylylendiurethan-2-methylethylenglykoldiurethandi(meth)acrylat (V380) weist die folgende Formel auf:

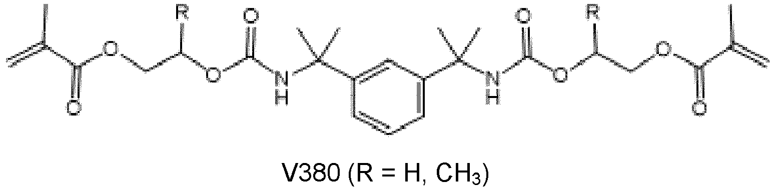

In der gezeigten Formel sind die Reste R unabhängig voneinander jeweils H oder CH₃, wobei die Reste die gleiche Bedeutung oder unterschiedliche Bedeutungen haben können. Vorzugsweise wird eine Mischung eingesetzt, die Moleküle, in denen beide Reste H sind, Moleküle, in denen beide Reste CH₃ sind, und Moleküle enthält, in denen ein Rest H und der andere Rest CH₃ ist, wobei das Verhältnis von H zu CH₃ vorzugsweise 7:3 ist. Eine solche Mischung ist beispielsweise durch Reaktion von 1,3-Bis(1-isocyanato-1-methylethyl)benzol mit 2-Hydroxypropylmethacrylat und 2-Hydroxyethylmethacrylat erhältlich.

Weitere bevorzugte difunktionelle Monomere sind radikalisch polymerisierbare Pyrrolidone, wie z.B. 1,6-Bis(3-vinyl-2-pyrrolidonyl)hexan, oder kommerziell zugängliche Bisacrylamide wie Methylen- oder Ethylenbisacrylamid, sowie Bis(meth)acrylamide, wie z.B. N,N'-Diethyl-1,3-bis(acrylamido)propan, 1,3-Bis(methacrylamido)-propan, 1,4-Bis(acrylamido)butan oder 1,4-Bis(acryloyl)piperazin, die durch Umsetzung aus den entsprechenden Diaminen mit (Meth)acrylsäurechlorid synthetisiert werden können. Besonders bevorzugt ist N,N'-Diethyl-1,3-bis(acrylamido)propan (V-392). Diese Monomere zeichnen sich durch eine hohe Hydrolysestabilität aus.

### Säuregruppenhaltige Monomere und Oligomere

Die erfindungsgemäßen Zusammensetzungen enthalten mindestens ein saures, radikalisch polymerisierbares Monomer und/oder mindestens ein saures Oligomer, wobei Zusammensetzungen, die mindestens ein saures, radikalisch polymerisierbares Monomer enthalten, bevorzugt sind. Unter sauren Monomeren und Oligomeren werden Monomere bzw. Oligomere verstanden, die mindestens eine Säuregruppe enthalten, vorzugsweise eine Phosphorsäureester-, Phosphonsäure- oder Carboxygruppe. Saure Monomere und Oligomere werden hierin auch als Haftkomponente bzw. Haftmonomere oder Haftoligomere bezeichnet. Erfindungsgemäß sich solche Zusammensetzungen besonders bevorzugt, die mindestens ein stark saures Monomer enthalten. Unter stark sauren Monomeren werden Monomere mit einem pKa-Wert von 0,5 bis 4,0, besonders bevorzugt 1,0 bis 3,5 und ganz besonders bevorzugt 1,5 bis 2,5 bei Raumtemperatur verstanden.

Geeignete säuregruppenhaltige Monomere sind COOH-gruppenhaltige polymerisationsfähige Monomere, vorzugsweise mit einem pKa-Wert im Bereich von 2,0 bis 4,0. Bevorzugt sind 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure. Methacrylsäure (pKa = 4,66) ist aufgrund ihrer geringen Haftung auf der Zahnhartsubstanz ausgeschlossen.

Bevorzugte säuregruppenhaltige Monomere sind Phosphorsäureester- und Phosphonsäuremonomere, vorzugsweise mit einem pKa-Wert im Bereich von 0,5 bis 3,5. Besonders bevorzugte sind 2-Methacryloyloxyethylphenylhydrogenphosphat, 10-Methacryloyloxydecyldihydrogenphosphat (MDP) Glycerindimethacrylatdihydrogenphosphat oder Dipentaerythritpentamethacryloyloxyphosphat. 4-Vinylbenzylphosphonsäure, 2-[4-(Dihydroxyphosphoryl)-2-oxa-butyl]-acrylsäure oder hydrolysestabile Ester, wie z.B. 2-[4-(Dihydroxyphosphoryl)-2-oxa-butyl]-acrylsäure-2,4,6-trimethylphenylester. Ganz besonders bevorzugt sind MDP, 2-Methacryloyloxyethylphenyl-hydrogenphosphat und Glycerindimethacrylatdihydrogenphosphat.

Unter Oligomeren werden Polymere mit einem Polymerisationsgrad Pₙ von 2 bis 100 verstanden (Pₙ = Mₙ / Mᵤ; Mₙ: zahlenmittlere Polymermolmasse, Mᵤ: Molmasse der Monomereinheit). Saure, radikalisch polymerisierbare Oligomere weisen mindestens eine Säuregruppe, vorzugsweise Carboxygruppe, und mindestens eine radikalisch polymerisierbare Gruppe, vorzugsweise mindestens eine (Meth)acrylat- und insbesondere mindestens eine Methacrylatgruppe, auf.

Erfindungsgemäß bevorzugte säuregruppenhaltige Oligomere sind oligomere Carbonsäuren, wie z.B. Polyacrylsäure, vorzugsweise mit einer zahlenmittleren Molmasse Mₙ kleiner 7200 g/mol, bevorzugt kleiner 7000 g/mol und besonders bevorzugt kleiner 6800 g/mol, wobei Mₙ bevorzugt in einem Bereich von 800 bis 7200 g/mol, besonders bevorzugt von 500 bis 7000 g/mol und ganz besonders bevorzugt von 500 bis 6800 g/mol liegt. Besonders bevorzugt sind oligomere Carbonsäuren mit (Meth)acrylatgruppen. Diese sind z.B. durch Umsetzung von oligomerer Polyacrylsäure mit Glycidylmethacrylat oder 2-Isocyanatoethylmethacrylat erhältlich.

Wenn nicht anders angegeben, handelt es sich hierin bei der Molmasse von Oligomeren und Polymeren um die zahlenmittlere Molmasse Mₙ, die mittels Gelpermeationschromatographie (GPC) bestimmt wird.

Die Gelpermeationschromatographie (GPC) ist eine Relativmethode bei der die Moleküle aufgrund ihrer Größe, genauer gesagt aufgrund ihres hydrodynamischen Volumens, getrennt werden. Die Bestimmung der absoluten Molmasse erfolgt durch Kalibrierung mit bekannten Standards. Als Kalibrierstandard werden vorzugsweise engverteile Polystyrolstandards eingesetzt. Diese sind kommerziell erhältlich. Als Trennmaterial werden Styrol-Divinylbenzol-Säulen und Tetrahydrofuran (THF) als Elutionsmittel verwendet. Styrol-Divinylbenzol-Säulen eignen sich für organische lösliche synthetische Polymere. Die Messung erfolgt mit verdünnten Lösungen (0,05 - 0,2 Gew.-%) der zu untersuchenden Polymere.

Alternativ kann die zahlenmittlere Molmasse mit den bekannten Methoden der Gefrierpunktserniedrigung (Kryoskopie), der Siedepunktserhöhung (Ebullioskopie) oder aus der Erniedrigung des Dampfdrucks (Dampfdruckosmometrie) bestimmt werden. Hierbei handelt es sich um absolute Methoden, die keine Kalibrierstandards erfordern. Es werden Konzentrationsreihen von 4 bis 6 verdünnten Polymerlösungen mit Konzentrationen von 0,005 bis 0,10 mol/kg untersucht und dann die gemessenen Werte auf eine Konzentration von 0 mol/kg extrapoliert.

Die erfindungsgemäßen Zusammensetzungen enthalten darüber hinaus vorzugsweise auch Wasser. Es wurde gefunden, dass ein Wassergehalt von 1 bis 7 Gew.-%, besonders bevorzugt 1 bis 5 Gew.-%, jeweils bezogen auf die Gesamtmasse der Zusammensetzung, eine Verbesserung der Haftwirkung an Dentin und Zahnschmelz bewirkt.

Die erfindungsgemäßen Zusammensetzungen enthalten außerdem mindestens einen Initiator zur Initiierung der radikalischen Polymerisation, vorzugsweise einen Photoinitiator. Bevorzugte Photoinitiatoren sind Benzophenon, Benzoin sowie deren Derivate, α-Diketone oder deren Derivate, wie 9,10-Phenanthrenchinon, 1-Phenyl-propan-1,2-dion, Diacetyl und 4,4'-Dichlorbenzil. Besonders bevorzugt werden Campherchinon (CC) und 2,2-Dimethoxy-2-phenyl-acetophenon und ganz besonders bevorzugt α-Diketone in Kombination mit Aminen als Reduktionsmittel, wie z.B. 4-(Dimethylamino)-benzoesäureethylester (EDMAB), N,N-Dimethylaminoethylmethacrylat, N,N-Dimethyl-sym.-xylidin oder Triethanolamin verwendet. Weiter bevorzugt sind Norrish-Typ-I-Photoinitiatoren, vor allem Acyl- oder Bisacylphosphinoxide und ganz besonders Monoacyltrialkyl-, Diacyldialkylgermanium- und Tetraacylgermanium-Verbindungen, wie z.B. Benzoyltrimethylgerman, Dibenzoyldiethylgerman, Bis(4-methoxybenzoyl)diethylgerman (Ivocerin^{®}), Tetrabenzoylgerman oder Tetrakis(o-methylbenzoyl)german. Dabei lassen sich auch Mischungen der verschiedenen Photoinitiatoren einsetzen, wie z.B. Bis(4-methoxybenzoyl)diethylgerman oder Tetrakis(o-methylbenzoyl)german in Kombination mit Campherchinon und 4-Dimethylaminobenzoesäureethylester.

Weiter bevorzugt sind Zusammensetzungen, die einen Redoxinitiator zur Initiierung der radikalischen Polymerisation enthalten, vorzugsweise einen Redoxinitiator auf der Basis eines Oxidationsmittels und eines Reduktionsmittel. Bevorzugte Oxidationsmittel sind Peroxide und insbesondere Hydroperoxide. Ein besonders bevorzugtes Peroxid ist Benzoylperoxid. Bevorzugte Hydroperoxide sind die in EP 3 692 976 A1 offenbarten geruchsarmen Cumolhydroperoxid-Derivate, die in EP 21315089.9 offenbarten oligomeren CHP-Derivate und insbesondere 4-(2-Hydroperoxypropan-2-yl)pheny¬propionat und Cumolhydroperoxid (CHP).

Bevorzugte Reduktionsmittel zur Kombination mit Peroxiden sind tertiäre Amine, wie N,N-Dimethyl-p-toluidin, N,N-Dihydroxyethyl-p-toluidin, p-Dimethylaminobenzoesäureethylester oder andere aromatische Dialkylamine, Ascorbinsäure, Sulfinsäuren, Thiole und/oder Hydrogensilane.

Bevorzugte Reduktionsmittel zur Kombination mit Hydroperoxiden sind Thioharnstoffderivate, insbesondere die in der EP 1 754 465 A1 in Absatz [0009] aufgeführten Verbindungen. Besonders bevorzugt sind Methyl-, Ethyl-, Allyl-, Butyl-, Hexyl-, Octyl-, Benzyl-, 1,1,3-Trimethyl-, 1,1-Diallyl, 1,3-Diallyl-, 1-(2-Pyridyl-2-thioharnstoff, Acetyl-, Propanoyl-, Butanoyl-, Pentanoyl-, Hexanoyl-, Heptanoyl-, Octanoyl, Nonanoyl, Decanoyl, Benzoylthioharnstoff und Mischungen davon. Ganz besonders bevorzugt sind Acetyl-, Allyl-, Pyridyl- und Phenylthioharnstoff sowie Hexanoylthioharnstoff und Mischungen davon sowie polymerisationsfähige Thioharnstoff-Derivate, wie z.B. *N*-(2-Methacryloyloxyethoxysuccinoyl)-thioharnstoff und *N*-(4-Vinylbenzoyl)-thioharnstoff). Außerdem kann vorteilhaft eine Kombination von einem oder mehreren der genannten Thioharnstoffderivate mit einem oder mehreren Imidazolen eingesetzt werden. Bevorzugte Imidazole sind 2-Mercapto-1-methylimidazol oder 2-Mercaptobenzimidazol.

Die erfindungsgemäßen Zusammensetzungen können neben mindestens einem Hydroperoxid und mindestens einem Thioharnstoffderivat außerdem zusätzlich mindestens eine Übergangsmetallverbindung zur Beschleunigung der Härtung enthalten. Erfindungsgemäß geeignete Übergangsmetallverbindungen sind insbesondere Verbindungen, die sich von Übergangsmetallen ableiten, die mindestens zwei stabile Oxidationsstufen haben. Besonders bevorzugt sind Verbindungen der Elemente Kupfer, Eisen, Kobalt, Nickel und Mangan. Diese Metalle weisen die folgenden stabilen Oxydationsstufen auf: Cu(I)/Cu(II), Fe(II)/Fe(III), Co(II)/Co(III), Ni(II)/Ni(III), Mn(II)/Mn(III). Zusammensetzungen, die mindestens eine Kupferverbindung enthalten, sind besonders geeignet. Die Übergansmetallverbindungen werden vorzugsweise in katalytische Mengen eingesetzt, besonders bevorzugt in einer Menge von 10 bis 200 ppm. Diese führen zu keiner Verfärbungen der Dentalmaterialien. Aufgrund der guten Monomerlöslichkeit werden die Übergangsmetalle vorzugsweise in Form ihrer Acetylacetonate, 2-Ethylhexanoate oder THF-Addukte eingesetzt. Bevorzugt sind weiterhin ihre Komplexe mit mehrzähnigen Liganden wie z.B. 2-(2-Amino-ethylamino)ethanol, Triethylentetramin, Dimethylglyoxim, 8-Hydroxychinolin, 2,2'-Bipyridin oder 1,10-Phenanthrolin. Ein erfindungsgemäß besonders geeigneter Initiator ist eine Mischung von Cumolhydroperoxid (CHP) mit mindestens einem der oben genannten Thioharnstoffderivate und Kupfer(II)-acetylacetonat. Erfindungsgemäß sind Zusammensetzungen bevorzugt, die keine Vanadiumverbindungen enthalten.

Die erfindungsgemäßen Zusammensetzungen können außerdem zusätzlich weitere Additive enthalten, vor allem Stabilisatoren, Farbmittel, mikrobizide Wirkstoffe, fluoridionenabgebende Additive, wie z.B. Fluorid-Salze, insbesondere NaF oder Ammoniumfluorid, oder Fluorsilane, optische Aufheller, Weichmacher und/oder UV-Absorber.

Erfindungsgemäß sind solche Zusammensetzungen bevorzugt, die die folgenden Bestandteile enthalten:
a) 25 bis 80 Gew.-%, bevorzugt 30 bis 75 Gew.-% und besonders bevorzugt 40 bis 70 Gew.-% mindestens eines beschichteten FAS- und/oder röntgenopaken Glasfüllstoffs,
b) 0 bis 50 Gew.-%, bevorzugt 0 bis 40 Gew.-% und besonders bevorzugt 2 bis 30 Gew.-% eines oder mehrerer weiterer Füllstoffe, vorzugsweise Metalloxide,
c) 1 bis 15 Gew.-%, bevorzugt 2 bis 12 Gew.-% und besonders bevorzugt 3 bis 10 Gew.-% mindestens eines säuregruppenhaltigen Monomers,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
e) 0 bis 10 Gew.-%, bevorzugt 0 bis 8 Gew.-% und besonders bevorzugt 1 bis 5 Gew.-% einer oder mehrerer oligomerer Carbonsäuren,
f) 0 bis 20 Gew.-%, bevorzugt 0 bis 15 Gew.-% und besonders bevorzugt 1 bis 10 Gew.-% eines oder mehrerer monofunktioneller Monomere ohne Säuregruppen,
g) 0,1 bis 8 Gew.-%, bevorzugt 0,5 bis 6 Gew.-% und besonders bevorzugt 1 bis 5 Gew.-% mindestens eines Initiators für die radikalische Polymerisation,
h) 0 bis 20 Gew.-%, bevorzugt 0,01 bis 10 Gew.-% und besonders bevorzugt 0,1 bis 7 Gew.-% Wasser und
i) 0,01 bis 5 Gew.-%, bevorzugt 0,1 bis 3 Gew.-% und besonders bevorzugt 0,1 bis 2 Gew.-% an Additiven, jeweils bezogen auf die Gesamtmasse der Zusammensetzung.

Der Initiator kann ein Redoxinitiator, ein Photoinitiator oder ein Initiator für die duale Härtung sein. Die genannten Mengenangaben enthalten sämtliche Initiatorbestandteile, d.h. die Initiatoren selbst und ggf. Reduktionsmittel, Übergangsmetallverbindung etc. Erfindungsgemäß sind Zusammensetzungen bevorzugt, die mindestens einen Redoxinitiator oder mindestens einen Redoxinitiator und mindestens einen Photoinitiator enthalten.

Zusammensetzungen, die einen Redoxinitiator enthalten, werden auch als selbsthärtend bezeichnet. Sie werden vorzugsweise in der Form von zwei räumlich getrennten Komponenten, d.h. als 2-Komponenten-System (2K System), eingesetzt. Oxidations- und Reduktionsmittel werden dabei in separate Komponenten der Zusammensetzung eingebarbeitet. Eine Komponente, die sog. Katalysatorpaste, enthält das Oxidationsmittel, vorzugsweise ein Peroxid oder Hydroperoxid, und ggf. einen Photoinitiator, und die zweite Komponente, die sog. Basen-Paste, enthält das entsprechende Reduktionsmittel, ggf. einen Photoinitiator und ggf. katalytische Mengen einer Übergansmetallverbindung. Die Polymerisation wird durch das Mischen der Komponenten gestartet. Zusammensetzungen, die sowohl einen Redox- als auch einen Photoinitiator enthalten, werden als dualhärtend bezeichnet.

Erfindungsgemäß sind 2-Komponenten-Systeme bevorzugt. Sie sind vorzugsweise selbsthärtend oder dualhärtend. Die Pasten werden kurz vor der Anwendung vorzugsweise mit einer Doppelschubspritze miteinander gemischt.

Die Katalysatorpaste weist vorzugsweise die folgende Zusammensetzung auf:
a) 25 bis 80 Gew.-%, bevorzugt 30 bis 75 Gew.-% und besonders bevorzugt 40 bis 70 Gew.-% mindestens eines beschichteten FAS- und/oder Glasfüllstoffs,
b) 0 bis 50 Gew.-%, bevorzugt 0 bis 40 Gew.-% und besonders bevorzugt 2 bis 30 Gew.-% eines oder mehrerer weiterer Füllstoffe, vorzugsweise Metalloxide,
c) 2 bis 30 Gew.-%, bevorzugt 4 bis 24 Gew.-% und besonders bevorzugt 6 bis 20 Gew.-% mindestens eines säuregruppenhaltigen Monomers,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
e) 0 bis 20 Gew.-%, bevorzugt 0 bis 16 Gew.-% und besonders bevorzugt 1 bis 10 Gew.-% einer oder mehrerer oligomerer Carbonsäuren,
f) 0 bis 20 Gew.-%, bevorzugt 0 bis 15 Gew.-% und besonders bevorzugt 1 bis 10 Gew.-% eines oder mehrerer monofunktioneller Monomere ohne Säuregruppen,
g) 0,01 bis 16 Gew.-%, bevorzugt 0,02 bis 12 Gew.-% und besonders bevorzugt 0,03 bis 10 Gew.-% mindestens eines Peroxids und/oder Hydroperoxids und ggf. mindestens eines Photoinitiators,
h) 0 bis 20 Gew.-%, bevorzugt 0,01 bis 10 Gew.-% und besonders bevorzugt 0,1 bis 7 Gew.-% Wasser und
i) 0,001 bis 5 Gew.-%, bevorzugt 0,002 bis 3 Gew.-% und besonders bevorzugt 0,0051 bis 2 Gew.-% eines oder mehrerer Additive, jeweils bezogen auf die Gesamtmasse der Katalysatorpaste.

Die Basen-Paste weist vorzugsweise folgende Zusammensetzung auf:
a) 25 bis 80 Gew.-%, bevorzugt 30 bis 75 Gew.-% und besonders bevorzugt 40 bis 70 Gew.-% mindestens eines FAS- und/oder Glasfüllstoffs,
b) 0 bis 50 Gew.-%. bevorzugt 0 bis 40 Gew.-% und besonders bevorzugt 2 bis 30 Gew.-% eines oder mehrerer weiterer Füllstoffe, vorzugsweise Metalloxide,
c) entfällt
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
e) entfällt
f) 0 bis 20 Gew.-%, bevorzugt 0 bis 15 Gew.-% und besonders bevorzugt 1 bis 10 Gew.-% eines oder mehrerer monofunktioneller Monomere ohne Säuregruppen,
g) 0,01 bis 16 Gew.-%, bevorzugt 0,02 bis 12 Gew.-% und besonders bevorzugt 0,1 bis 10 Gew.-% mindestens eines geeigneten Reduktionsmittels und ggf. mindestens eines Photoinitiators,
h) 0 bis 20 Gew.-%, bevorzugt 0,01 bis 10 Gew.-% und besonders bevorzugt 0,1 bis 7 Gew.-% Wasser und
i) 0,001 bis 5 Gew.-%, bevorzugt 0,002 bis 3 Gew.-% und besonders bevorzugt 0,005 bis 2 Gew.-% eines oder mehrerer Additive, jeweils bezogen auf die Gesamtmasse der Basen-Paste.

Die Beschichtung der Füllstoffe verhindert Wechselwirkungen der Füllstoffe mit sauren Monomeren oder Oligomeren. Saure Monomere und Oligomere sind erfindungsgemäß nur in der Katalysatorpaste enthalten, so dass zur Herstellung der Basen-Paste vorzugsweise unbeschichtete FAS-Füllstoffe und/oder röntgenopake Glasfüllstoffe eingesetzt werden, wobei die oben beschriebenen FAS-Füllstoffe und/oder röntgenopake Glasfüllstoffe in unbeschichteter Form bevorzugt sind.

Zur Anwendung werden die Katalysator- und Basen-Paste vorzugsweise in etwa gleichen Anteilen miteinander gemischt. Sie eignen sich daher besonders zur Anwendung mit einer Doppelschubspritze.

Doppelschubspritzen weisen zwei getrennte zylindrische Kammern zur Aufnahme von Basis- und Katalysatorpaste auf. Die Komponenten werden mit zwei miteinander verbundenen Kolben gleichzeitig aus den Kammern herausgedrückt und vorzugsweise durch eine Mischkanüle gepresst und darin miteinander gemischt. Zum Herausdrücken der Pasten kann die Spritze in einen sogenannten Handdispenser eingesetzt werden, der die Handhabung der Spritzen erleichtert.

Die erfindungsgemäßen Zusammensetzungen zeichnen sich durch eine hohe Lagerstabilität und eine verbesserte Transparenz, vorzugsweise größer 10 %, und gute Selbsthaftung auf Schmelz/Dentin aus. Sie eignen sich besonders als Dentalwerkstoffe zur intraoralen Anwendung durch den Zahnarzt zur Restauration geschädigter Zähne (therapeutische Anwendung), insbesondere als dentale Zemente, Beschichtungs- oder Verblendmaterialien, Füllungskomposite und ganz besonders als Befestigungszemente. Die Transparenz wird auf die in den Ausführungsbeispielen beschriebene Weise bestimmt.

Durch die Beschichtung ist es möglich, mit herkömmlichen röntgenopaken Füllstoffen, die die Herstellung von Kompositen mit einer hohen Transparenz ermöglichen aber aufgrund ihrer Säureempfindlichkeit nachteilig sind, stabile dentale Komposite herzustellen. Es war zu erwarten, dass bei der Beschichtung der Füllstoffe oberflächliche Rest-Silanol-Gruppen (Si-OH), die sich aus den Silanen der Formel I bilden, zu Wasserstoffbrückennetzwerken führen und damit eine deutliche Erhöhung der Viskosität bewirken. Es wurde überraschend gefunden, dass die erfindungsgemäße Beschichtung zu keiner Erhöhung der Verdickungswirkung der Füllstoffe führt. Außerdem hat sich gezeigt, dass die beschichteten Füllstoffe die Mischbarkeit von Katalysator- und Basis-Paste verbessern und so die Herstellung homogener Materialien erheblich erleichtern.

Zur Behandlung geschädigter Zähne werden diese vorzugsweise in einem ersten Schritt durch den Zahnarzt präpariert. Anschließend wird mindestens eine erfindungsgemäße Zusammensetzung auf oder in den präparierten Zahn appliziert. Danach kann die Zusammensetzung direkt gehärtet werden, vorzugsweise durch Bestrahlen mit Licht einer geeigneten Wellenlänge, beispielsweise bei der Versorgung von Kavitäten. Alternativ wird eine Dentalrestauration, beispielsweise ein Inlay, Onlay, Veneer, eine Krone, Brücke, ein Gerüst oder eine Dentalkeramik, in den präparierten Zahn eingebracht oder darauf aufgebracht. Die anschließende Härtung der Zusammensetzung erfolgt vorzugsweise durch Licht und/oder durch Selbsthärtung. Die Dentalrestauration wird hierbei am Zahn befestigt.

Die erfindungsgemäßen Zusammensetzungen können auch als extraorale Werkstoffe (nicht therapeutisch) eingesetzt werden, beispielsweise bei der Herstellung oder Reparatur von Dentalrestaurationen. Sie eignen sich zudem als Materialien zur Herstellung und Reparatur von Inlays, Onlays, Kronen oder Brücken.

Zur Herstellung von Dentalrestaurationen wie Inlays, Onlays, Kronen oder Brücken, wird mindestens eine erfindungsgemäße Zusammensetzung auf an sich bekannte Weise zu der gewünschten Dentalrestauration geformt und dann gehärtet. Die Härtung kann durch Licht, Selbsthärtung oder vorzugsweise thermisch erfolgen.

Bei der Reparatur von Dentalrestaurationen werden die erfindungsgemäßen Zusammensetzungen auf die zu reparierende Restauration aufgebacht, beispielsweise um Lücken auszubessern oder um Bruchstücke zu verkleben, und anschließend gehärtet.

Die Erfindung wird im Folgenden anhand von Figuren und Beispielen näher erläutert.

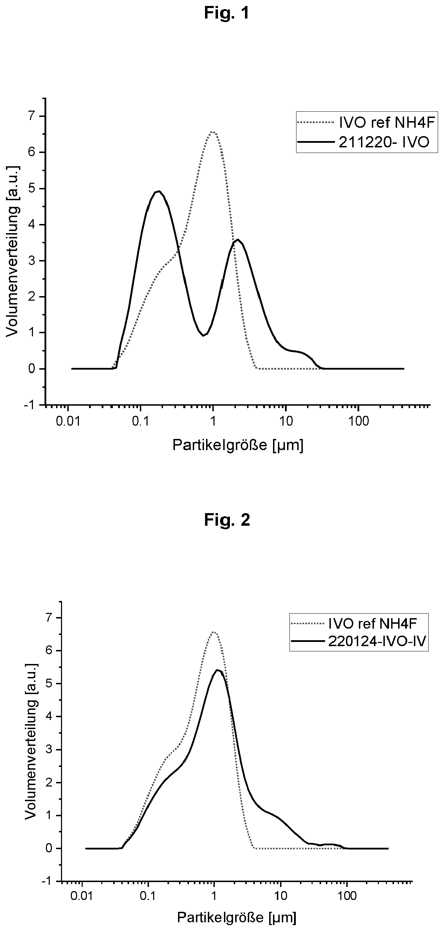

Die Figuren 1 bis 3 zeigen jeweils die Partikelgrößenverteilung erfindungsgemäß beschichteter Füllstoffe im Vergleich zu den unbeschichteten Ausgangsmaterialien.

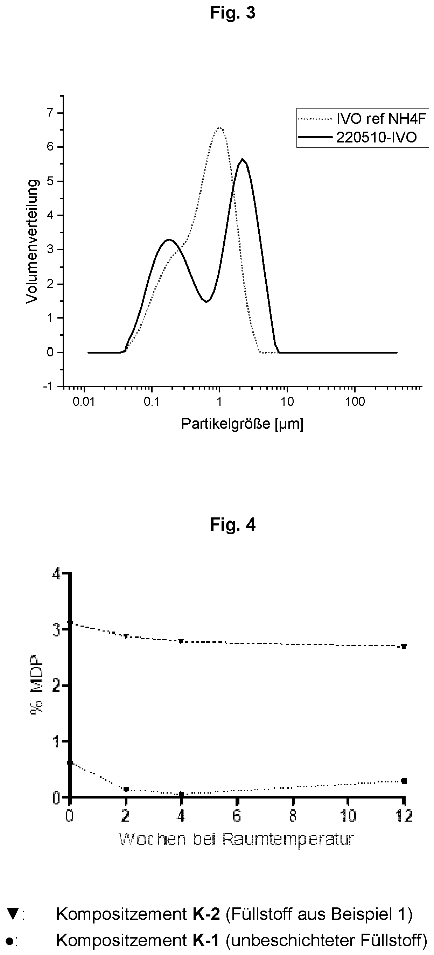

Figur 4 zeigt die Abnahme der Konzentration des sauren Monomers MDP in Abhängigkeit von der Lagerzeit in einer Kompositpaste mit dem beschichteten Glasfüllstoff aus Beispiel 1 (--▼--) im Vergleich zu einer Kompositpaste mit dem unbeschichteten Glasfüllstoff (--■--). In der Paste mit dem unbeschichteten Füllstoff nimmt die MDP-Konzentration rasch ab, während die erfindungsgemäße Paste über den Untersuchungszeitraum stabil ist.

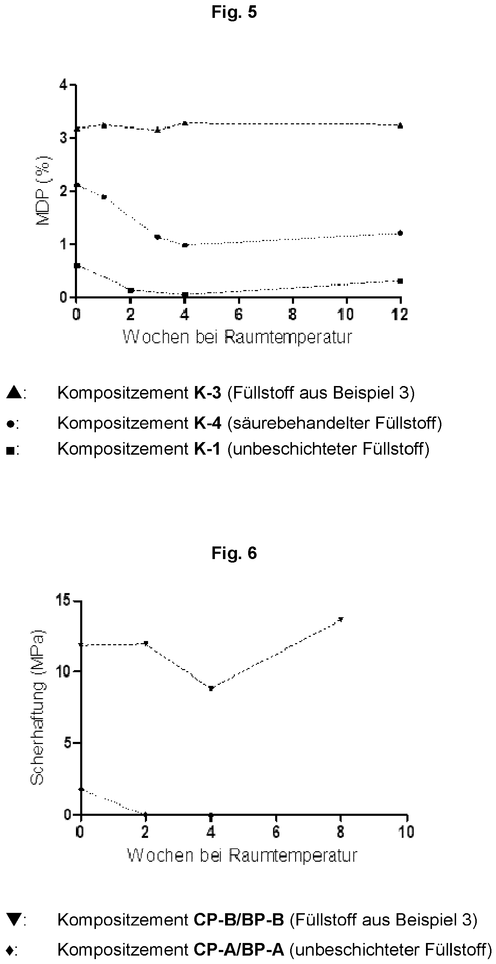

Figur 5 zeigt die Abnahme der Konzentration des sauren Monomers MDP in Abhängigkeit von der Lagerzeit in einer Kompositpaste mit dem beschichteten Glasfüllstoff aus Beispiel 3 (--▲--) im Vergleich zu einer Kompositpaste mit dem unbeschichteten Glasfüllstoff (--■--) und einer Kompositpaste mit einem säurebehandelten Füllstoff (--●--). Die Paste mit dem säurebehandelten Füllstoff weist gegenüber der Paste mit dem unbeschichteten Füllstoff eine deutlich höhere Stabilität auf. Die Stabilität der erfindungsgemäßen Paste mit dem beschichteten Füllstoffe ist nochmals erheblich verbessert. Über den Untersuchungszeitraum wurde keine Abnahme der MDP-Konzentration beobachtet.

Figur 6 zeigt die Dentin-Scherhaftfestigkeit eines zweikomponentigen Kompositzements mit dem beschichteten Füllstoff aus Beispiel 1 (obere Kurve) im Vergleich zu einem Zement mit unbeschichtetem Füllstoff (untere Kurve) in Abhängigkeit von deren Lagerungszeit. Der erfindungsgemäße Zement erzielt auch nach mehrwöchiger Lagerung hohe Haftwerte, während der Vergleichszement bereits nach zwei Wochen keine Haftwirkung mehr bewirkt.

### Ausführungsbeispiele

### Beispiel 1:

### Herstellung eines beschichteten Glasfüllers (Feststoffgehalt 10 Gew.-%)

Eine Mischung von 120,0 g des röntgenopaken Glasfüllstoffs GM 27884, (Schott AG; mittlere Partikelgröße 1 µm; spezifische Oberfläche (BET DIN ISO 9277) 3,9 m²/g; Zusammensetzung (Gew.-%): Al₂O₃: 10, B₂O₃: 10, BaO: 25 und SiO₂: 55), 1080,0 g des Lösungsmittels Isopropoxyethanol (IPE), 750,0 g Tetraethoxysilan (TEOS) und 376,0 g deionisiertes Wasser wurden in einem 5 L Doppelwanderaktor mit KPG-Rührer und Rückflusskühler mit einer Rührgeschwindigkeit von 500 Umdrehungen pro Minute (U/min) bei 23°C gerührt. Der Feststoffgehalt betrug 10 Gew.-%. Zur Herstellung einer Katalysator-Lösung wurden 0,325 g Ammoniumfluorid in 250 mL deionisiertem Wasser gelöst und über Nacht gerührt. 12,83 g dieser NH₄F-Lösung wurden zu der obigen Reaktionsmischung zugetropft. Anschließend wurde der Ansatz auf 100°C erwärmt und für 3 Stunden bei 100°C weiter gerührt. Danach wurde die Mischung in vier 500 ml-Zentrifugenbehälter verteilt und für 15 Minuten bei 2000 Umdrehungen pro Minute zentrifugiert. Die abgetrennten Füllstoffpartikel wurden mindestens zweimal mit Isopropanol gewaschen und erneut zentrifugiert. Anschließend wurden die gewaschen Füllstoffpartikel für 12 Stunden bei 60°C im Vakuumtrockenschrank getrocknet. Die erhaltene Probe hat die Bezeichnung: 211220-IVO.

Zur Bestimmung der Partikelgrößenverteilung wurden 0,1 g der beschichteten Partikel (Probe 211220-IVO) in 50 g deionisiertes Wasser gegeben und zwei Stunden auf einem Magnetrührer gerührt. Anschließend wurde die Probe 30 Minuten im Ultraschallbad behandelt. Parallel dazu wurden 0,1 g unbeschichtete Füllstoffpartikel (Probe IVO ref NH₄F) auf die beschriebene Weise in 50 g Wasser dispergiert. Der Füllstoff IVO ref NH₄F wurde auf die oben beschriebene Weise hergestellt, allerdings wurde kein Silan (TEOS) zugegeben. Die Partikelgrößenverteilung wurde mittels dynamischer Lichtstreuung (DLS) bestimmt (Malvern Mastersizer 2000 mit Dispergiereinheit Hydro 2000S). Die Partikelgrößenverteilung (Partikelgrößenverteilungshistogramm) ist in Figur 1 wiedergegeben. Für die beschichtete Probe 211220-IVO wurde eine bimodale Partikelgrößenverteilung mit Peak-Maxima bei 0,2 µm und 2 µm bestimmt.

Der Brechungsindex des eingesetzten Füllstoffs (n_{D} = 1,526) wurde durch die Beschichtung nicht verändert. Die Brechungsindices des beschichteten und des unbeschichteten Füllstoffs wurden mit der Immersionsmethode bestimmt (Carl-Zeiss Abbe Refraktometer Modell A 72901; Immersionsöle: 2-Hydroxy-3-phenoxypropyl-acrylat (n_{D} = 1,526) und Zedernöl (n_{D} = 1,504)).

### Beispiel 2:

### Bestimmung der Ionenfreisetzung der Füllstoffe aus Beispiel 1 in Säure

Zur Bestimmung der Barrierewirkung wurden 2 g der beschichteten Füllstoffpartikel aus Bsp. 1 (Probe 211220-IVO) in 50 g 1 M HCl dispergiert und 1 h gerührt. Nach dem Abzentrifugieren der ausgelaugten Partikel wurde der Al-, B, Ba-Gehalt des Zentrifugats bestimmt. Hierzu wurden die Proben abhängig von der Al-, B, Ba-IonenKonzentration in der Lösung im Verhältnis von 1:10 oder 1:100 mit Reinstwasser (Millli-Q^{®}, Firma Merck) verdünnt. Die Analyse wurde mittels Optischer Emissionsspektroskopie mit induktiv gekoppeltem Plasma durchgeführt (ICP-OES, Inductively Coupled Plasma Optical Emission Spectrometry; Messgerät: Horiba Jobin Yvon UItima2 mit Conikal-Zerstäuber; Messparameter: Druck: 2,71 bar, Durchfluss: 0,73 l/min, Emissionswellenlängen: AI: λ = 396,152 nm, B: λ = 208,957 nm, Ba: λ = 455,403 nm). Es wurden die folgenden Konzentrationen bestimmt: Al: 3,97 mg/L; B: 2,74 mg/L und Ba: 23,0 mg/L.

Demgegenüber ergab ein analoger Auslaugeversuch mit dem nichtbeschichteten Füllstoff IVO ref NH₄F folgende Konzentrationen: Al: 226,9 mg/L; B: 126,7 mg/L und Ba: 1060 mg/L.

Ein Vergleich der Werte zeigt, dass durch die Beschichtung der Füllstoffe die Auslaugung von Al-Ionen um 98,2 Gew.-% und von Ba-Ionen um 97,8 Gew.-% verringert wurde.

### Beispiel 3:

### Herstellung eines beschichteten Glasfüllers mit reduzierter Silan-Konzentration

Eine Mischung von 120,0 g des röntgenopaken Glasfüllstoffs GM 27884, 1080,0 g IPE, 250,0 g TEOS und 125,3 g deionisiertem Wasser wurde in einem 5 L Doppelwandreaktor mit einer Rührgeschwindigkeit von 500 U/Min. bei 23°C gerührt. Der Feststoffgehalt betrug 10 Gew.-%. 4,28 g der NH₄F-Lösung aus Beispiel 1 wurden zu Reaktionsmischung zugetropft, der Ansatz anschließend auf 100°C erwärmt und für 24 Stunden bei 100°C gerührt. Danach wurde die Mischung wie in Beispiel 1 beschrieben zentrifugiert, die abgetrennten Füllstoffpartikel gewaschen und getrocknet (Probe 220124-IVO). Die Partikelgrößenverteilung wurde analog zu Beispiel bestimmt und ist in der Figur 2 dargestellt. Die DLS Messung zeigte eine monomodale Partikelgrößenverteilung, die weitgehend der der unbeschichteten Referenzprobe IVO ref NH₄F entsprach. Das Peak-Maximum lag bei ca. 4 µm.

Der Brechungsindex des eingesetzten Füllstoffs (n_{D} = 1,526) wurde durch die Beschichtung nicht verändert.

Der Auslaugeversuch analog zu Beispiel 2 ergab folgende Konzentrationen: Al: 1,75 mg/L; B: 1,43 mg/L und Ba: 18,87 mg/L. Die Auslaugung von Al-Ionen wurde durch die Beschichtung um 99,2 Gew.-% und die von Ba-Ionen um 98,2 Gew.-% verringert, jeweils im Vergleich zu der Referenzprobe IVO ref NH₄F.

### Beispiel 4:

### Herstellung eines beschichteten Glasfüllers (Feststoffgehalt 20 Gew.-%)

Eine Mischung von 150,0 g des röntgenopaken Glasfüllstoffs GM 27884, 600,0 g IPE, 312,50 g TEOS und 156,66 g deionisiertem Wasser wurde in einem 5 L Doppelwandreaktor mit einer Rührgeschwindigkeit von 500 U/Min. bei 23°C gerührt. 5,35 g der NH₄F-Lösung aus Beispiel 1 werden zu Reaktionsmischung zugetropft, der Ansatz anschließend auf 100°C erwärmt und für 24 Stunden bei 100°C gerührt. Danach wurde die Mischung wie in Beispiel 1 beschrieben zentrifugiert, die abgetrennten Füllstoffpartikel gewaschen und getrocknet (Probe 220510-IVO). Die Partikelgrößenverteilung wurde analog zu Beispiel 1 bestimmt und ist in der Figur 3 dargestellt. Die DLS Messung zeigte eine bimodale Partikelgrößenverteilung mit Peak-Maxima bei 0,2 µm und 2,2 µm, ähnlich wie die Probe 211220-IVO in Beispiel 1.

Der Auslaugeversuch analog zu Beispiel 2 ergab folgende Konzentrationen: Al: 5,067 mg/L; B: 1,91 mg/L und Ba: 10,49 mg/L. Die Auslaugung von Al-Ionen wurde durch die Beschichtung um 97,8 Gew.-% und die von Ba-Ionen um 99,0 Gew.-% verringert, jeweils im Vergleich zu der Referenzprobe IVO ref NH₄F.

### Beispiel 5:

### Herstellung von säurebehandelten Füllstoffen (allgemeine Vorschrift gemäß EP 21218194.5)

In zwei Zentrifugenkunststoffgefäßen mit einem Füllvolumen von jeweils 1 L werden je 150 g des zu behandelten Füllstoffs und 350 g 1,0 molare Salzsäure eingefüllt und 1 h bei Raumtemperatur auf einer Magnetrührplatte gerührt. Nach dem Entfernen der Magnetrührer werden die Mischungen in einer Zentrifuge (Hettich Silenta RS) für 5 min bei 3000 Umdrehungen pro min zentrifugiert, wobei sich der Füllstoff absetzt. Die Flüssigkeit wird abgetrennt und eine Probe der Flüssigkeit für die Röntgenfluoreszens (XRF)-Analyse genommen. Der pH-Wert der Flüssigkeit liegt bei 1-2. Anschließend wird der abgetrennte Füllstoff in 400 ml deionisiertem Wasser dispergiert und die Dispersion erneut für 5 min bei 3000 Umdrehungen pro min zentrifugiert. Dann wird die Waschlösung durch Dekantieren abgetrennt. Der Waschvorgang wird so oft wiederholt, bis der pH-Wert im letzten Waschvorgang auf 5 oder mehr ansteigt (ca. dreimal). Nach dem Waschen wird der säurebehandelte Füllstoff Im Vakuumtrockenschrank bei 50°C bis zur Gewichtskonstanz getrocknet und dann silanisiert. Zur Silanisierung wird der Füllstoff (185 g) mit 12 g 3-Methacryloyloxypropyltrimethoxysilan (Silan A-174, Sigma Aldrich) versetzt und anschließend für 15 min gemischt (Turbola-Mischer, Willy A. Bachofen AG). Danach werden 5 g deionisiertes Wasser zugegeben und nochmals für 15 min gemischt. Anschließend wird der Füllstoff durch ein 90-µm-Kunststoffsieb gesiebt, für 24 h ruhen gelassen und dann für 3 Tage im Trockenschrank bei 50°C getrocknet bis kein freies Silan mehr nachweisbar ist (Gaschromatographie).

### Beispiel 6:

### Bestimmung der Lagerstabilität erfindungsgemäß beschichteter Glasfüller

Es wurden die Kompositzementpasten **K-1** bis **K-4** mit den in Tabelle 1 angegebenen Zusammensetzungen hergestellt:

**Tabelle 1: Zusammensetzung von Kompositpasten [Gew.-%]**

| **Bestandteil** | **K-1*)** | **K-2** | **K-3** | **K-4*)** |
|---|---|---|---|---|
| unbeschichteter Glasfüllstoff¹⁾ | 65 | - | - | - |
| beschichteter Glasfüllstoff aus Bsp. 1 | - | 65 | - | - |
| beschichteter Glasfüllstoff aus Bsp. 3 | - | - | 65 | - |
| säurebehandelter Glasfüllstoff²⁾ | - | - | - | 65 |
| HDK 2000³⁾ | 4 | 4 | 4 | 4 |
| MDP⁴⁾ | 3,29 | 3,29 | 3,29 | 3,29 |
| TEGDMA⁵⁾ | 8,53 | 8,53 | 8,53 | 8,53 |
| NK-Ester 9G⁶⁾ | 1,90 | 1,90 | 1,90 | 1,90 |
| V-392⁷⁾ | 11,68 | 11,68 | 11,68 | 11,68 |
| Wasser | 5,58 | 5,58 | 5,58 | 5,58 |
| BHT⁸⁾ | 0,03 | 0,03 | 0,03 | 0,03 |

| | | | | |
|---|---|---|---|---|
| *) Vergleichsbeispiel ¹⁾ nichtbehandelter Glasfüllstoff GM 27884, 1,0 µm, unsilanisiert (Schott AG) ²⁾ Glasfüllstoff GM 27884,1,0 µm, gemäß Bsp. 5 mit Säure behandelt ³⁾ Pyrogene Kieselsäure (spezifische Oberfläche (BET) 120 m²/g, Wacker AG) ⁴⁾ 10-Methacryloyloxydecyldihydrogenphosphat (Orgentis) ⁵⁾ Triethylenglycoldimethacrylat ⁶⁾ Polyethylenglycol-400-dimethacrylat (Kowa Europa GmbH) ⁷⁾ N,N'-Diethyl-1,3-bis(acrylamido)-propan (Ivoclar Vivadent AG) ⁸⁾ 2,6-Di-*tert*-butyl-*p*-kresol | | | | |

Die Pasten wurden für einen Zeitraum von mehreren Wochen bei Raumtemperatur gelagert und wiederholt der Gehalt des sauren Monomers MDP mittels HPLC bestimmt. Für die HPLC-Messung wurde das Gerät HPLC Ultimate 3000 (ThermoFisher Scientific) mit einer Säule 125x4 Nucleodur 100-5 C18ec mit UV/VIS-Detektor (220 nm) verwendet. Die zu messenden Proben wurden in Methanol gelöst. Die Elution erfolgte mit den Eluenten Wasser mit 0,01 mol/L o-Phosphorsäure und Acetonitril gemäß einem üblichen Gradientenprogramm (der Acetonitrilanteil wurde kontinuierlich auf 100% gesteigert). Die Ergebnisse sind in den Figuren 4 und 5 dargestellt. und zeigen die zeitliche Änderung des Gehalts an MDP in den Kompositpasten.

Die in Figur 4 dargestellten Ergebnisse belegen eine deutlich verbesserte Lagerstabilität des Kompositzements **K-2** im Vergleich zu dem Kompositzement **K-1** mit dem unbeschichteten Glasfüller GM 27884. **K1** zeigt eine deutliche Abnahme des verfügbaren MDPs schon nach 2 bis 4 Wochen. Die Unterschiede zum Zeitpunkt 0 sind dadurch bedingt, dass das saure Monomer bei der Pastenherstellung sehr schnell mit dem unbehandelten Füllstoff reagiert.

Die in Figur 5 dargestellten Ergebnisse zeigen eine deutlich verbesserte Lagerstabilität des Kompositzements **K-4,** der einen säurebehandelten Glasfüller gemäß der europäischen Patentanmeldung Nr. EP 21218194.5 enthält, im Vergleich zu dem Kompositzement **K-1** mit unbeschichtetem Füller. Der erfindungsgemäße Kompositzement **K-3** zeigt eine nochmals verbesserte Stabilität. Auch nach Lagerung für 12 Wochen ist keine Änderung der MDP-Konzentration feststellbar.

### Beispiel 7:

### Messung der Dentin-Scherhaftfestiakeit erfindungsgemäßer Kompositzemente

Es wurden dualhärtende zwei-Komponenten Kompositzemente hergestellt. Jeder Zement umfasste eine Katalysator- und eine Basispaste. Die Zusammensetzungen der Pasten sind in den Tabellen 2 und 3 angegeben. Es wurde die Dentinhaftung in Abhängigkeit von der Lagerzeit bestimmt. Für die Messung der Dentinhaftung wurden Rinderzähne mit einem additionsvernetzenden Vinyl-Polysiloxan (Firma Dreve) so in einen Kunststoffzylinder eingebettet, dass sich das Dentin und der Kunststoff in einer Ebene befanden. Die mit Schleifpapier (Körnung 400) geschliffenen Zahnoberflächen wurden mit handwarmem Wasser gespült und auf 37°C vortemperiert. Die Dentinoberflächen wurden trockengetupft, die Katalysatorpasten jeweils mit der zugehörigen Basispaste im Verhältnis 1:1 gemischt und anschließend auf die Zahnoberflache aufgebracht. Gleichzeitig wurde die Unterseite eines Pfropfens aus einem gehärteten dentalen Kompositmaterial (Tetric Evo-Ceram, Fa. Ivoclar Vivadent AG) mit dem Zement benetzt und möglichst mittig auf die Dentinoberfläche platziert. Anschließend wurde der Zahn mit dem Komposit-Pfropfen nach oben in einer Ultradent-Einspannvorrichtung so eingespannt, dass der Fixierungsdorn mittig auf dem Tetric-Evo-Ceram-Pfropfen aufsaß. Danach wurde der Überschuss an Befestigungszement sofort sorgfältig entfernt und die Ultradent-Einspannvorrichtung mit dem eingespannten Zahn für 15 Minuten bei 37°C im Trockenschrank gelagert. Danach wurden die Pfropfen entlastet, für 24 Stunden in Wasser bei 37°C aufbewahrt und dann für 24 Stunden im Trockenschrank bei 37°C gelagert. Zur Messung der Scherhaftung wurden die Pfropfen mittels einer Zwick-Prüfmaschine nach der Ultradent-Methode (EN ISO 35 29022, Jahr 2013) bei 23°C abgeschert, wobei sich die Scherhaftfestigkeit als Quotient der Bruchkraft und der Haftfläche ergab. Die Ergebnisse sind in Figur 6 dargestellt.

**Tabelle 2: Zusammensetzung der Katalysatorpasten CP-A und CP-B**

| **Bestandteil** | **CP-A*** | **CP-B** |
|---|---|---|
| MDP⁴⁾ | 3,41 | 3,41 |
| TEGDMA⁵⁾ | 10,72 | 10,72 |
| NK-Ester 9G⁶⁾ | 2,38 | 2,38 |
| UDMA¹⁴⁾ | 14,68 | 14,68 |
| DBPO¹⁷⁾ | 0,97 | 0,97 |
| BHT⁸⁾ | 0,031 | 0,031 |
| Wasser | 0,831 | 0,827 |
| unbeschichteter Glasfüllstoff¹⁾ | 63,43 | - |
| Füller aus Bsp. 3 | - | 63,43 |
| HDK 2000³⁾ | 2,10 | 2,11 |
| HDK H-15¹⁶⁾ | 1,44 | 1,44 |

| | | |
|---|---|---|
| * Vergleichsbeispiel | | |

**Tabelle 3: Zusammensetzung der Basenpasten BP-A und BP-B**

| **Bestandteil** | **BP-A** | **BP-B** |
|---|---|---|
| TEGDMA⁵⁾ | 8,62 | 8,54 |
| NK-Ester 9G⁶⁾ | 2,53 | 2,51 |
| UDMA¹⁴⁾ | 12,60 | 12,50 |
| DABA¹⁵⁾ | 0,600 | 1,502 |
| Campherchinon | 0,076 | 0,075 |
| 4-Dimethylaminobezoesäureethylester | 0,152 | 0,151 |
| Phasentransferkatalysator⁹⁾ | 0,203 | 0,201 |
| Farbpigment¹⁰⁾ | 0,00074 | 0,00076 |
| TEMPO¹¹⁾ | 0,00077 | 0,00080 |
| Wasser | 0,6501 | 0,644 |
| Glasfüller G018-056, 7 µm, 5 % silan.¹²⁾ | 69,98 | 69.64 |
| Glasfüller G018-056, 1 µm, 5 % silan.¹³⁾ | 3,08 | 3,05 |
| HDK 2000³⁾ | 1,49 | 1,48 |

| | | |
|---|---|---|
| ¹⁾ nichtbehandelter Glasfüllstoff GM 27884, 1,0 µm, unsilanisiert (Schott AG) ³⁾ Pyrogene Kieselsäure (spezifische Oberfläche (BET) 120 m²/g, Wacker AG) ⁴⁾ 10-Methacryloyloxydecyldihydrogenphosphat (Orgentis) ⁵⁾ Triethylenglycoldimethacrylat ⁶⁾ Polyethylenglycol-400-dimethacrylat (Kowa Europa GmbH) ⁸⁾ 2,6-Di-*tert*-butyl-*p*-kresol ⁹⁾ Benzyltributylammoniumchlorid ¹⁰⁾ 2,5-Dihydroxyterephthalsäurediethylester (Lumilux LZ Flu Blau, Riedel-de Haën AG) ¹¹⁾ 2,2,6,6-Tetramethylpiperidinyloxyl (CAS-Nummer 2564-83-2) ¹²⁾ Glasfüller G018-056, 7 µm, 5 % silan.: 24 Gew.-% Al₂O₃, 23 Gew.-% SiO₂, 16,5 Gew.-% CaO, 16 Gew.-% CaF₂, 11,5 Gew.-% BaO, 8 Gew.-% P₂O₅, 2 Gew.-% Na₂O, 5% Silan; gewichtsmittlerer Partikeldurchmesser 7 µm (Schott AG, Mainz) ¹³⁾ Glasfüller G018-056, 1 µm, 5 % silan.: 24 Gew.-% Al₂O₃, 23 Gew.-% SiO₂, 16,5 Gew.-% CaO, 16 Gew.-% CaF₂, 11,5 Gew.-% BaO, 8 Gew.-% P₂O₅, 2 Gew.-% Na₂O, 5% Silan; gewichtsmittlerer Partikeldurchmesser 1 µm (Schott AG, Mainz) ¹⁴⁾ 1,6-Bis-[2-methacryloyloxyethoxycarbonylamino]-2,2,4-trimethylhexan ¹⁵⁾ N,N-Diethyl-3,5-di-tert.-butylanilin ¹⁶⁾ Pyrogene Kieselsäure (spezifische Oberfläche (BET) 130 - 170 m²/g, Wacker AG) ¹⁷⁾ 50 % Dibenzoylperoxid (DBPO) dispergiert in 50 % Dicyclohexylphthalat (DBPO: BP 50 FT; Fa. United Initiator) | | |

Die in Figur 6 dargestellten Ergebnisse zeigen, dass der erfindungsgemäße Zement **CP-B/BP-B,** der durch Mischen der Katalysatorpaste CP-B und der Basenpaste BP-B erhalten wurde, auch nach Lagerung über mehrere Wochen hohe Haftwerte erzielt. Die erfindungsgemäße Katalysatorpaste CP-B enthält das stark saure Monomer MDP und einen erfindungsgemäß beschichteten, röntgenopaken Ba-Glasfüllstoff. Die gemessenen Haftwerte belegen, dass die Beschichtung des Füllstoffs unerwünschte Reaktionen mit dem sauren Monomer wirksam unterdrückt. Die Basenpasten enthalten keine sauren Monomere, so dass hier unbeschichtete Glasfüllstoffe eingesetzt wurden. Der Vergleichszement **CP-A/BP-A** mit unbeschichtetem Füllstoff zeigte schon nach Lagerung für nur 2 Wochen keine Haftung mehr.

## Patentansprüche

1. Radikalisch polymerisierbare Zusammensetzung, die mindestens ein polyfunktionelles radikalisch polymerisierbares Monomer ohne Säuregruppe, mindestens ein säuregruppenhaltiges radikalisch polymerisierbares Monomer und/oder saures Oligomer, mindestens einen Fluoroaluminiumsilikatglasfüllstoff und/oder röntgenopaken Glasfüllstoff und mindestens einen Initiator für die radikalische Polymerisation enthält, **dadurch gekennzeichnet, dass** der Fluoroaluminiumsilikatglasfüllstoff und/oder röntgenopake Glasfüllstoff mit einem Kieselsäure(hetero)polykondensat beschichtet ist.

2. Zusammensetzung nach Anspruch 1, bei der der Fluoroaluminiumsilikatglasfüllstoff und/oder röntgenopake Glasfüllstoff eine Beschichtung aufweist, die durch hydrolytische Kondensation einer oder mehrerer Silane der allgemeinen Formel I
SiXₐR₄₋ₐ Formel I
in der die Variablen die folgenden Bedeutungen haben:
R = C₁-C₂₀-Alkyl oder C₂-C₁₀-Alkenyl, wobei Alkyl- und Alkenyl-Reste ein oder mehrere O- oder S-Atome, Ester-, Amid-, Amino- oder Urethan-Gruppen enthalten können, C₆-C₁₄-Aryl oder C₆-C₁₅-Arylalkyl,
X = Halogen, Hydroxy, C₁-C₁₀-Alkoxy-, C₁-C₆-Acyloxy;
a = 2, 3 oder 4, vorzugsweise 3 oder 4.
und gegebenenfalls einer oder mehrerer weiterer hydrolytisch kondensierbarer Verbindungen des Bors, Aluminiums, Titans und/oder Phosphors und/oder von den voranstehend genannten Verbindungen abgeleiteten Vorkondensaten erhältlich ist.

3. Zusammensetzung nach Anspruch 2, bei der die Menge an Silanen der Formel I 10 bis 100 Gew.-% bezogen auf die Gesamtmenge an hydrolytisch kondensierbaren Verbindungen beträgt.

4. Zusammensetzung nach einem der vorhergehenden Ansprüche, bei der der beschichtete Fluoroaluminiumsilikatglasfüllstoff und/oder röntgenopake Glasfüllstoff dadurch erhältlich ist, dass man den zu beschichtenden Füllstoff in einem oder mehreren Silanen der Formel I und ggf. weiteren hydrolytisch kondensierbaren Verbindungen oder vorzugsweise einer Lösung des oder der Silane der Formel I und ggf. weiterer hydrolytisch kondensierbaren Verbindungen in einem geeigneten Lösungsmittel dispergiert, und man dann Wasser zugibt, die resultierende Mischung rührt, dann den Füllstoff abtrennt und trocknet.

5. Zusammensetzung nach einem der vorhergehenden Ansprüche, die
einen röntgenopaken Glasfüllstoff mit der Zusammensetzung (Gew.-%): SiO₂: 20-80; B₂O₃: 5-15; BaO oder SrO: 0-30; Al₂O₃: 5-20; CaO und/oder MgO: 0-20; Na₂O, K₂O, Cs₂O: je 0-10; WO₃: 0-20; La₂O₃: 0-10; ZrO₂: 0-15; P₂O₅: 0-10; Ta₂O₅, Nb₂O₅ oder Yb₂O₃: 0-5 und CaF₂ oder SrF₂: 0-10, oder vorzugsweise SiO₂: 50-75; B₂O₃: 5-15; BaO oder SrO: 2-30; Al₂O₃: 5-15; CaO und/oder MgO: 0-10 und Na₂O: 0-10,
und/oder
einen Fluoroaluminiumsilikatglasfüllstoff mit der Zusammensetzung (Gew.-%): SiO₂: 20-35; Al₂O₃: 15-35; BaO oder SrO: 0-25; CaO: 0-20; ZnO: 0-15; P₂O₅: 5-20; Fluorid 3-18, oder vorzugsweise SiO₂: 20-30; Al₂O₃: 20-30; BaO oder SrO: 5-15; CaO: 5-18; P₂O₅: 5-15; Na₂O: 2-10; und CaF₂: 5-20,
enthält, wobei sich alle Angaben auf die Gesamtmasse des Glases beziehen und wobei alle Komponenten mit Ausnahme von Fluor als Oxide berechnet werden.

6. Zusammensetzung nach einem der vorhergehenden Ansprüche, bei der das polyfunktionelle radikalisch polymerisierbare Monomer aus Bisphenol-A-dimethacrylat, Bis-GMA (ein Additionsprodukt aus Methacrylsäure und Bisphenol-A-diglycidylether), ethoxy- oder propoxyliertes Bisphenol-A-dimethacrylaten, wie Bisphenol-A-dimethacrylat mit 3 Ethoxygruppen oder 2,2-Bis[4-(2-methacryloyloxypropoxy)phenyl]propan, UDMA (ein Additionsprodukt aus 2-Hydroxyethylmethacrylat und 2,2,4-Trimethylhexamethylen-1,6-diisocyanat), Tetramethylxylylendiurethanethylenglycoldi(meth)acrylat bzw. Tetramethylxylylendiurethan-2-methylethylenglykoldiurethandi(meth)acrylat (V380), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryloyloxymethyl)tricyclo-[5.2.1.0^{2,6}]decan (DCP), Polyethylenglycol- oder Polypropylenglycoldimethacrylaten, wie Polyethylenglycol-200-dimethacrylat (PEG-200-DMA) oder Polyethylenglycol-400-dimethacrylat (PEG-400-DMA), 1,12-Dodecandioldimethacrylat, Urethanen aus 2-(Hydroxymethyl)acrylsäure und Diisocyanten, wie 2,2,4-Trimethylhexamethylendiisocyanat oder Isophorondiisocyanat, Pyrrolidonen, wie z.B. 1,6-Bis(3-vinyl-2-pyrrolidonyl)-hexan, Bisacrylamiden, wie Methylen- oder Ethylenbisacrylamid, Bis(meth)-acrylamiden, wie N,N'-Diethyl-1,3-bis(acrylamido)-propan, 1,3-Bis(methacryl-amido)propan, 1,4-Bis(acrylamido)-butan oder 1,4-Bis(acryloyl)-piperazin, und Mischungen davon ausgewählt ist.

7. Zusammensetzung nach einem der vorhergehenden Ansprüche, die als säuregruppenhaltiges Monomer mindestens ein Monomer enthält, das aus Monomeren, die eine Phosphorsäureestergruppe oder Phosphonsäuregruppe enthalten, vorzugsweise 2-Methacryloyloxyethylphenylhydrogenphosphat, 10-Methacryloyloxydecyldihydrogenphosphat (MDP) Glycerindimethacrylatdihydrogenphosphat, Dipentaerythritpentamethacryloyloxyphosphat, 4-Vinylbenzylphosphonsäure, 2-[4-(Dihydroxyphosphoryl)-2-oxa-butyl]-acrylsäure und/oder 2-[4-(Dihydroxyphosphoryl)-2-oxa-butyl]-acrylsäure-2,4,6-trimethylphenylester, und/oder 4-(Meth)acryloyloxyethyltrimellitsäureanhydrid, 10-Methacryloyloxydecylmalonsäure, N-(2-Hydroxy-3-methacryloyloxypropyl)-N-phenylglycin und/oder 4-Vinylbenzoesäure ausgewählt ist.

8. Zusammensetzung nach einem der Ansprüche 4 bis 7, die als saures Oligomer eine Polyacrylsäure mit einer zahlenmittleren Molmasse kleiner 7200 g/mol, bevorzugt kleiner 7000 g/mol und besonders bevorzugt kleiner 6800 g/mol enthält.

9. Zusammensetzung nach einem der vorhergehenden Ansprüche, die zusätzlich mindestens ein monofunktionelles radikalisch polymerisierbares Monomer ohne Säuregruppe enthält, das aus Benzyl-, Tetrahydrofurfuryl(meth)acrylat, Isobornyl(meth)acrylat, p-Cumyl-phenoxyethylenglycolmethacrylat (CMP-1E) und 2-([1,1'-Biphenyl]-2-oxy)ethylmethacrylat (MA-836), Tricyclodecanmethyl(meth)acrylat, 2-(2-Biphenyloxy)ethyl(meth)acrylat, 2-Hydroxyethyl-(methacrylat), Hydroxyethylpropyl(methacrylat), 2-Acetoxyethylmethacrylat und Mischungen davon ausgewählt ist.

10. Zusammensetzung nach einem der vorhergehenden Ansprüche, die
a) 25 bis 80 Gew.-%, bevorzugt 30 bis 75 Gew.-% und besonders bevorzugt 40 bis 70 Gew.-% mindestens eines beschichteten Fluoroaluminiumsilikatglasfüllstoffs und/oder röntgenopaken Glasfüllstoffs,
b) 0 bis 50 Gew.-%, bevorzugt 0 bis 40 Gew.-% und besonders bevorzugt 2 bis 30 Gew.-% eines oder mehrerer weitere Füllstoffe, vorzugsweise Metalloxide,
c) 1 bis 15 Gew.-%, bevorzugt 2 bis 12 Gew.-% und besonders bevorzugt 3 bis 10 Gew.-% mindestens eines säuregruppenhaltigen Monomers,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
e) 0 bis 10 Gew.-%, bevorzugt 0 bis 8 Gew.-% und besonders bevorzugt 1 bis 5 Gew.-% einer oder mehrerer oligomerer Carbonsäuren,
f) 0 bis 20 Gew.-%, bevorzugt 0 bis 15 Gew.-% und besonders bevorzugt 1 bis 10 Gew.-% eines oder mehrerer monofunktioneller Monomere ohne Säuregruppen,
g) 0,1 bis 8 Gew.-%, bevorzugt 0,5 bis 6 Gew.-% und besonders bevorzugt 1 bis 5 Gew.-% mindestens eines Initiators für die radikalische Polymerisation,
h) 0 bis 20 Gew.-%, bevorzugt 0,01 bis 10 Gew.-% und besonders bevorzugt 0,1 bis 7 Gew.-% an Wasser und
i) 0,01 bis 5 Gew.-%, bevorzugt 0,1 bis 3 Gew.-% und besonders bevorzugt 0,1 bis 2 Gew.-% an Additiven
enthält, jeweils bezogen auf die Gesamtmasse der Zusammensetzung.

11. Zusammensetzung nach einem der vorhergehenden Ansprüche, die eine Katalysatorpaste und eine Basen-Paste umfasst, wobei die Katalysatorpaste
a) 25 bis 80 Gew.-%, bevorzugt 30 bis 75 Gew.-% und besonders bevorzugt 40 bis 70 Gew.-% mindestens eines beschichteten Fluoroaluminiumsilikatglasfüllstoffs und/oder Glasfüllstoffs,
b) 0 bis 50 Gew.-%, bevorzugt 0 bis 40 Gew.-% und besonders bevorzugt 2 bis 30 Gew.-% eines oder mehrerer weiterer Füllstoffe, vorzugsweise Metalloxide,
c) 2 bis 30 Gew.-%, bevorzugt 4 bis 24 Gew.-% und besonders bevorzugt 6 bis 20 Gew.-% mindestens eines säuregruppenhaltigen Monomers,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
e) 0 bis 20 Gew.-%, bevorzugt 0 bis 16 Gew.-% und besonders bevorzugt 1 bis 10 Gew.-% einer oder mehrerer oligomerer Carbonsäuren,
f) 0 bis 20 Gew.-%, bevorzugt 0 bis 15 Gew.-% und besonders bevorzugt 1 bis 10 Gew.-% eines oder mehrerer monofunktioneller Monomere ohne Säuregruppen,
g) 0,01 bis 16 Gew.-%, bevorzugt 0,02 bis 12 Gew.-% und besonders bevorzugt 0,03 bis 10 Gew.-% mindestens eines Peroxids und/oder Hydroperoxids und ggf. mindestens eines Photoinitiators,
h) 0 bis 20 Gew.-%, bevorzugt 0,01 bis 10 Gew.-% und besonders bevorzugt 0,1 bis 7 Gew.-% Wasser und
i) 0,001 bis 5 Gew.-%, bevorzugt 0,002 bis 3 Gew.-% und besonders bevorzugt 0,0051 bis 2 Gew.-% eines oder mehrerer Additive enthält, jeweils bezogen auf die Gesamtmasse der Katalysatorpaste,
und wobei die Basen-Paste
a) 25 bis 80 Gew.-%, bevorzugt 30 bis 75 Gew.-% und besonders bevorzugt 40 bis 70 Gew.-% mindestens eines FAS- und/oder Glasfüllstoffs,
b) 0 bis 50 Gew.-%. bevorzugt 0 bis 40 Gew.-% und besonders bevorzugt 2 bis 30 Gew.-% eines oder mehrerer weiterer Füllstoffe, vorzugsweise Metalloxide,
c) entfällt
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
e) entfällt
f) 0 bis 20 Gew.-%, bevorzugt 0 bis 15 Gew.-% und besonders bevorzugt 1 bis 10 Gew.-% eines oder mehrerer monofunktioneller Monomere ohne Säuregruppen,
g) 0,01 bis 16 Gew.-%, bevorzugt 0,02 bis 12 Gew.-% und besonders bevorzugt 0,1 bis 10 Gew.-% mindestens eines geeigneten Reduktionsmittels und ggf. mindestens eines Photoinitiators,
h) 0 bis 20 Gew.-%, bevorzugt 0,01 bis 10 Gew.-% und besonders bevorzugt 0,1 bis 7 Gew.-% Wasser und
i) 0,001 bis 5 Gew.-%, bevorzugt 0,002 bis 3 Gew.-% und besonders bevorzugt 0,005 bis 2 Gew.-% eines oder mehrerer Additive enthält, jeweils bezogen auf die Gesamtmasse der Basen-Paste.

12. Zusammensetzung nach einem der vorhergehenden Ansprüche zur therapeutischen Anwendung als Dentalwerkstoff, vorzugsweise als dentaler Zement, Beschichtungs- oder Verblendmaterial, Füllungskomposit oder Befestigungszement.

13. Nicht-therapeutische Verwendung einer Zusammensetzung gemäß einem der Ansprüche 1 bis 11 zur Herstellung oder Reparatur von Dentalrestaurationen, insbesondere von Inlays, Onlays, Kronen oder Brücken.

14. Verfahren zur Herstellung eines beschichteten Fluoroaluminiumsilikatglasfüllstoffs oder Glasfüllstoffs, bei dem man
(i) den zu beschichtenden Füllstoff in einem oder mehreren Silanen der Formel I und ggf. weiteren hydrolytisch kondensierbaren Verbindungen oder vorzugsweise einer Lösung des oder der Silane der Formel I und ggf. weiterer hydrolytisch kondensierbarer Verbindungen in einem geeigneten Lösungsmittel dispergiert,
(ii) man dann Wasser zugibt,
(iii) man die resultierende Mischung rührt,
(iv) man dann den Füllstoff abtrennt und trocknet.

15. Verwendung eines Fluoroaluminiumsilikatglasfüllstoffs oder Glasfüllstoffs, der gemäß Anspruch 14 herstellbar ist, zur Stabilisierung dentaler, radikalisch polymerisierbarer Zusammensetzungen.
